# EUROPEAN PATENT SPECIFICATION

(11) **EP 3 185 888 B1**
(45) Date of publication and mention of the grant of the patent: **07.06.2023**
(21) Application number: 15836120.4
(22) Date of filing: 25.08.2015
(51) Int. Cl.: A61K 38/39, A61P 1/02, A61P 1/04, A61P 13/02, A61P 13/10, A61P 17/00, A61P 19/04, A61P 25/00, A61P 27/16

(54) **EXTRACELLULAR MATRIX COMPOSITIONS**
EXTRAZELLULÄRE MATRIXZUSAMMENSETZUNGEN
COMPOSITIONS DE MATRICE EXTRACELLULAIRE

(30) Priority: 25.08.2014 US 201462041468 P
(43) Date of publication of application: 05.07.2017
(73) Proprietor: Celularity Inc., Florham Park, NJ 07932 (US)
(72) Inventor: BHATIA, Mohit, B., Manalapan, NJ 07726 (US); HERZBERG, Uri, Bridgewater, NJ 08807 (US); KAPLUNOVSKY, Aleksandr, Budd Lake, NJ 07828 (US); ZAKA, Raihana, Randolph, NJ 07869 (US); YE, Qian, Livingston, NJ 07039 (US)
(74) Representative: Mewburn Ellis LLP
(86) International application number: PCT/US2015/046690
(87) International publication number: WO 2016/033041

(56) References cited:
- WO-A1-2014/039427
- WO-A1-2014/089440
- WO-A1-2015/123183
- WO-A2-2010/135527
- US-A1- 2004 037 813
- US-A1- 2009 028 817
- US-A1- 2009 228 105
- US-A1- 2012 141 595
- US-A1- 2014 011 743
- US-B2- 7 875 273
- BHATIA ET AL.: 'Placenta Derived Adherent Cell (PDAC) Interaction and Response on Extracellular Matrix Isolated from Human Placenta' WOUNDS vol. 20, no. 2, 01 February 2008, pages 1 - 8, XP009500563

## Description

### 1. FIELD

Provided herein are extracellular matrix (ECM) compositions and methods of making the same. Also provided herein are uses of the ECM compositions provided herein.

### 2. BACKGROUND

Extracellular matrix (ECM) comprises proteins that form many structures in the body, including tendons, ligaments, and sheets that support skin and internal organs. There remains a need in the art for new and improved ECM compositions and methods of making such ECM compositions.

### 3. SUMMARY

The invention is as defined in the claims. Extracellular matrix (ECM) compositions may be prepared using placental tissue, e.g., human placental tissue.

ECM compositions described herein may comprise about 30% to about 60% collagen and about 10% to about 35% elastin. In addition, such ECM compositions comprise (i) very low amounts of fibronectin *(e.g.,* less than 0.1% fibronectin), *e.g.,* as measured by ELISA; (ii) no or an undetectable amount of laminin, e.g., as measured by ELISA; and/or no or an undetectable amount of glycosaminoglycans, e.g., as measured by ELISA.

ECM compositions described herein may comprise about 30% to about 72% collagen and about 10% to about 35% elastin. In addition, such ECM compositions can comprise (i) fibronectin *(e.g.,* less than 0.1% fibronectin), *e.g.,* as measured by ELISA; (ii) laminin *(e.g.,* less than 0.1% laminin), *e.g.,* as measured by ELISA; glycosaminoglycans, (*e.g.,* less than 0.1% glycosaminoglycans) e.g., as measured by ELISA; (iii) no or an undetectable amount of cytokines; (iv) no or an undetectable amount of growth factors; and/or (v) no or an undetectable amount of deoxycholic acid.

The ECM compositions provided herein comprise characteristics that make them well-suited for therapeutic/medical use. Specifically, the ECM compositions described herein are sterile, acellular *(e.g.,* ≥99% cell-free) and/or are free of cellular debris *(e.g.,* ≥99% free of cellular debris). In particular embodiments, the ECM compositions provided herein comprise no undetectable cytokines or an undetectable amount of cytokines, as measured by, *e.g.,* ELISA. In certain embodiments, the ECM compositions provided herein further are devoid of reagent residuals, *i.e.,* the final ECM compositions comprise undetectable amounts of reagents used in the manufacture of the compositions. Further, in particular embodiments, the ECM compositions provided herein comprise minimal amounts of nucleic acid *(e.g.,* ~41-171 ng/mg of dry product) and endotoxin (e.g., <0.25 EU).

Another advantageous characteristic of the ECM compositions provided herein is their ability to absorb water. ECM compositions described herein may absorb between 150%-225% their weight in water. Such a characteristic is advantageous in, e.g., wound healing applications of the ECM compositions provided herein.

In a specific embodiment provided herein is an ECM composition comprising 35-55% collagen by dry weight and 10-30% elastin by dry weight. In a specific embodiment, said composition comprises 34-53% collagen and 13-29% elastin. In another specific embodiment, said ECM composition is obtained from placental tissue, e.g., human placental tissue. In another specific embodiment, said ECM composition is obtained from the chorion of a placenta, *e.g.,* the chorion from a human placenta. In another specific embodiment, said ECM composition comprises a very low amount of fibronectin, e.g., less than 0.1% fibronectin, less than 0.01% fibronectin, or less than 0.001% fibronectin. In another specific embodiment, said ECM composition comprises an undetectable amount of laminin. In another specific embodiment, said ECM composition comprises an undetectable amount of glycosaminoglycans. In another specific embodiment, said ECM composition comprises a very low amount of fibronectin, e.g., less than 0.1% fibronectin, less than 0.01% fibronectin, or less than 0.001% fibronectin; no or an undetectable amount of laminin; and an undetectable amount of glycosaminoglycans.

Described herein but not explicitly claimed is an ECM composition comprising about 35-72% collagen and about 15-25% elastin. In specific embodiment provided herein is an ECM composition comprising 40-70% collagen by dry weight and 15-25% elastin by dry weight. In another specific embodiment, said composition comprises 40-70% collagen and 15-22% elastin. In yet another specific embodiment, said composition comprises 43-68% collagen and 18-21% elastin. In certain embodiments, said ECM composition comprises fibronectin, e.g., less than 0.1% fibronectin, less than 0.05% fibronectin, less than 0.01% fibronectin, less than 0.001% fibronectin, 0.001 to 0.1% fibronectin, 0.001 to 0.05% fibronectin, 0.001 to 0.01% fibronectin, 0.01 to 0.1% fibronectin, or 0.01 to 0.05% fibronectin. In another specific embodiment, said ECM composition comprises laminin, e.g., less than 0.1% laminin, less than 0.05% laminin, less than 0.01% laminin, less than 0.001% laminin, 0.001 to 0.1% laminin, 0.001 to 0.05% laminin, 0.001 to 0.01% laminin, 0.01 to 0.1% laminin, or 0.01 to 0.05% laminin. In another specific embodiment, said ECM composition comprises glycosaminoglycans, e.g., less than 0.1% glycosaminoglycans, less than 0.05% glycosaminoglycans, less than 0.01% glycosaminoglycans, less than 0.001% glycosaminoglycans, 0.001 to 0.1% glycosaminoglycans, 0.001 to 0.05% glycosaminoglycans, 0.001 to 0.01% glycosaminoglycans, 0.01 to 0.1% glycosaminoglycans, or 0.01 to 0.05% glycosaminoglycans. In another specific embodiment, said ECM composition comprises an undetectable amount of cytokines, growth factors, and/or deoxycholic acid. In certain embodiments, said ECM composition comprises fibronectin, e.g., less than 0.1% fibronectin, less than 0.05% fibronectin, less than 0.01% fibronectin, less than 0.001% fibronectin, 0.001 to 0.1% fibronectin, 0.001 to 0.05% fibronectin, 0.001 to 0.01% fibronectin, 0.01 to 0.1% fibronectin, or 0.01 to 0.05% fibronectin, laminin, e.g., less than 0.1% laminin, less than 0.05% laminin, less than 0.01% laminin, less than 0.001% laminin, 0.001 to 0.1% laminin, 0.001 to 0.05% laminin, 0.001 to 0.01% laminin, 0.01 to 0.1% laminin, or 0.01 to 0.05% laminin, glycosaminoglycans, e.g., less than 0.1% glycosaminoglycans, less than 0.05% glycosaminoglycans, less than 0.01% glycosaminoglycans, less than 0.001% glycosaminoglycans, 0.001 to 0.1% glycosaminoglycans, 0.001 to 0.05% glycosaminoglycans, 0.001 to 0.01% glycosaminoglycans, 0.01 to 0.1% glycosaminoglycans, or 0.01 to 0.05% glycosaminoglycans, and, additionally, comprises an undetectable amount of cytokines, growth factors, and/or deoxycholic acid. In another specific embodiment, said ECM composition is obtained from placental tissue, e.g., human placental tissue. In another specific embodiment, said ECM composition is obtained from the chorion of a placenta, for example the chorionic plate of a placenta, *e.g.,* the chorion, for example the chorionic plate, from a human placenta.

Described herein is an ECM composition comprising about 50-60% collagen and about 10-20% elastin. Said ECM may be composition may be obtained from placental tissue, e.g., human placental tissue. Said ECM composition may be obtained from the chorion of a placenta, *e.g.,* the chorion from a human placenta. Said ECM composition may comprise a very low amount of fibronectin, e.g., less than 0.1% fibronectin, less than 0.01% fibronectin, or less than 0.001% fibronectin. Said ECM composition may comprise no or an undetectable amount of laminin. Said ECM composition may comprise no or an undetectable amount of glycosaminoglycans. Said ECM composition may comprise a very low amount of fibronectin, e.g., less than 0.1% fibronectin, less than 0.01% fibronectin, or less than 0.001% fibronectin; no or an undetectable amount of laminin; and no or an undetectable amount of glycosaminoglycans.

Described herein is an ECM composition comprising about 45-55% collagen and about 15-25% elastin. Said ECM composition may be obtained from placental tissue, e.g., human placental tissue. Said ECM composition may be obtained from the chorion of a placenta, *e.g.,* the chorion from a human placenta. Said ECM composition may comprise a very low amount of fibronectin, e.g., less than 0.1% fibronectin, less than 0.01% fibronectin, or less than 0.001% fibronectin. Said ECM composition may comprise no or an undetectable amount of laminin. Said ECM composition may comprises no or an undetectable amount of glycosaminoglycans. Said ECM composition may comprise a very low amount of fibronectin, e.g., less than 0.1% fibronectin, less than 0.01% fibronectin, or less than 0.001% fibronectin; no or an undetectable amount of laminin; and no or an undetectable amount of glycosaminoglycans.

Described herein is an ECM composition comprising about 40-50% collagen and about 20-30% elastin. Said ECM may be composition may be obtained from placental tissue, e.g., human placental tissue. Said ECM composition may be obtained from the chorion of a placenta, *e.g.,* the chorion from a human placenta. Said ECM composition may comprise a very low amount of fibronectin, e.g., less than 0.1% fibronectin, less than 0.01% fibronectin, or less than 0.001% fibronectin. Said ECM composition may comprise no or an undetectable amount of laminin. Said ECM may composition may comprise no or an undetectable amount of glycosaminoglycans. Said ECM composition may comprise a very low amount of fibronectin, e.g., less than 0.1% fibronectin, less than 0.01% fibronectin, or less than 0.001% fibronectin; no or an undetectable amount of laminin; and no or an undetectable amount of glycosaminoglycans.

Described herein, but not explicitly claimed, is an ECM composition comprising about 30-40% collagen and about 25-35% elastin. Said ECM may be composition may be obtained from placental tissue, e.g., human placental tissue. Said ECM composition may be obtained from the chorion of a placenta, *e.g.,* the chorion from a human placenta. Said ECM composition may comprise a very low amount of fibronectin, e.g., less than 0.1% fibronectin, less than 0.01% fibronectin, or less than 0.001% fibronectin. Said ECM composition may comprise no or an undetectable amount of laminin. Said ECM composition may comprises no or an undetectable amount of glycosaminoglycans. Said ECM composition may comprise a very low amount of fibronectin, *e.g.,* less than 0.1% fibronectin, less than 0.01% fibronectin, or less than 0.001% fibronectin; no or an undetectable amount of laminin; and no or an undetectable amount of glycosaminoglycans.

Described herein is an ECM composition comprising about 34-43% collagen and about 16-24% elastin. Said ECM composition may be obtained from placental tissue, *e.g.,* human placental tissue. Said ECM composition may be obtained from the chorion of a placenta, *e.g.,* the chorion from a human placenta. Said ECM composition may comprise a very low amount of fibronectin, *e.g.,* less than 0.1% fibronectin, less than 0.01% fibronectin, or less than 0.001% fibronectin. Said ECM composition may comprise no or an undetectable amount of laminin. Said ECM composition may comprise no or an undetectable amount of glycosaminoglycans. Said ECM compositiont may comprise a very low amount of fibronectin, *e.g.,* less than 0.1% fibronectin, less than 0.01% fibronectin, or less than 0.001% fibronectin; no or an undetectable amount of laminin; and no or an undetectable amount of glycosaminoglycans.

Described herein is an ECM composition comprising about 37-42% collagen and about 16-24% elastin. Said ECM composition may be obtained from placental tissue, *e.g.,* human placental tissue. Said ECM composition may be obtained from the chorion of a placenta, *e.g.,* the chorion from a human placenta. Said ECM composition may comprise a very low amount of fibronectin, *e.g.,* less than 0.1% fibronectin, less than 0.01% fibronectin, or less than 0.001% fibronectin. Said ECM composition may comprises no or an undetectable amount of laminin. Said ECM composition may comprise no or an undetectable amount of glycosaminoglycans. Said ECM composition may comprise a very low amount of fibronectin, *e.g.,* less than 0.1% fibronectin, less than 0.01% fibronectin, or less than 0.001% fibronectin; no or an undetectable amount of laminin; and no or an undetectable amount of glycosaminoglycans.

Described herein is an ECM composition comprising about 30-70%, 30-60%, 30-50%, 30-40%, 30-35%, 34-43%, 35-40%, 37-42%, 40-70%, 40-60%, 40-50%, 40-45%, 40-65%, 43-68%, 45-50%, 50-55%, or 55-60% collagen and about 10-30%, 10-20%, 10-15%, 15-25%, 15-22%, 15-20%, 16-24%, 17-24%, 18-21%, 18-20%, 20-24%, 20-30%, 20-25%, or about, 25-30%, elastin. Said ECM may be composition may be obtained from placental tissue, *e.g.,* human placental tissue. Said ECM composition may be obtained from the chorion of a placenta, for example the chorionic plate, *e.g.,* the chorion, for example, the chorionic plate, from a human placenta. Said ECM composition may be comprise a very low amount of fibronectin, *e.g.,* less than 0.1% fibronectin, less than 0.05% fibronectin, less than 0.01% fibronectin, or less than 0.001% fibronectin. Said ECM composition may comprise 0.001 to 0.1% fibronectin, 0.001 to 0.05% fibronectin, 0.001 to 0.01% fibronectin, 0.01 to 0.1% fibronectin, or 0.01 to 0.05% fibronectin. Said ECM composition may comprise no or an undetectable amount of laminin. Said ECM composition may comprise a very low amount of laminin, *e.g.,* less than 0.1% laminin, less than 0.05% laminin, less than 0.01% laminin, or less than 0.001% laminin. Said ECM composition may comprise laminin, *e.g.,* 0.001 to 0.1% laminin, 0.001 to 0.05% laminin, 0.001 to 0.01% laminin, 0.01 to 0.1% laminin, or 0.01 to 0.05% laminin. Said ECM composition may comprise no or an undetectable amount of glycosaminoglycans. Said ECM composition may comprise a very low amount of glycosaminoglycans, *e.g.,* less than 0.1% glycosaminoglycans, less than 0.05% glycosaminoglycans, less than 0.01% glycosaminoglycans, or less than 0.001% glycosaminoglycans. Said ECM composition may comprise glycosaminoglycans, *e.g.,* 0.001 to 0.1% glycosaminoglycans, 0.001 to 0.05% glycosaminoglycans, 0.001 to 0.01% glycosaminoglycans, 0.01 to 0.1% glycosaminoglycans, or 0.01 to 0.05% glycosaminoglycans. Said ECM composition may comprise a very low amount of fibronectin, *e.g.,* less than 0.1% fibronectin, less than 0.01% fibronectin, or less than 0.001% fibronectin; no or an undetectable amount of laminin; and no or an undetectable amount of glycosaminoglycans. Said ECM composition may comprises- fibronectin, *e.g.,* less than 0.1% fibronectin, less than 0.05% fibronectin, less than 0.01% fibronectin, less than 0.001% fibronectin, 0.001 to 0.1% fibronectin, 0.001 to 0.05% fibronectin, 0.001 to 0.01% fibronectin, 0.01 to 0.1% fibronectin, or 0.01 to 0.05% fibronectin, laminin, *e.g.,* less than 0.1% laminin, less than 0.05% laminin, less than 0.01% laminin, less than 0.001% laminin, 0.001 to 0.1% laminin, 0.001 to 0.05% laminin, 0.001 to 0.01% laminin, 0.01 to 0.1% laminin, or 0.01 to 0.05% laminin, glycosaminoglycans, *e.g.,* less than 0.1% glycosaminoglycans, less than 0.05% glycosaminoglycans, less than 0.01% glycosaminoglycans, less than 0.001% glycosaminoglycans, 0.001 to 0.1% glycosaminoglycans, 0.001 to 0.05% glycosaminoglycans, 0.001 to 0.01% glycosaminoglycans, 0.01 to 0.1% glycosaminoglycans, or 0.01 to 0.05% glycosaminoglycans, and, additionally, may comprise no or an undetectable amount of cytokines, growth factors, and/or deoxycholic acid.

Described herein is an ECM composition comprising about 30%, about 35%, about 40%, about 45%, about 50%, about 55%, about 60%, about 65%, or about 70% collagen and about 10%, about 15%, about 18%, about 20%, about 25%, or about 30%, elastin. Said ECM composition may be obtained from placental tissue, *e.g.,* human placental tissue. Said ECM composition may be obtained from the chorion of a placenta, for example, the chorionic plate, *e.g.,* the chorion, for example, the chorionic plate, from a human placenta. Said ECM composition may comprise a very low amount of fibronectin, *e.g.,* less than 0.1% fibronectin, less than 0.05% fibronectin, less than 0.01% fibronectin, or less than 0.001% fibronectin. Said ECM composition may comprise 0.001 to 0.1% fibronectin, 0.001 to 0.05% fibronectin, 0.001 to 0.01% fibronectin, 0.01 to 0.1% fibronectin, or 0.01 to 0.05% fibronectin. Said ECM composition may comprises no or an undetectable amount of laminin. Said ECM composition may comprise a very low amount of laminin, *e.g.,* less than 0.1% laminin, less than 0.05% laminin, less than 0.01% laminin, or less than 0.001% laminin. Said ECM composition may comprise laminin, *e.g.,* 0.001 to 0.1% laminin, 0.001 to 0.05% laminin, 0.001 to 0.01% laminin, 0.01 to 0.1% laminin, or 0.01 to 0.05% laminin. Said ECM composition may comprise no or an undetectable amount of glycosaminoglycans. Said ECM composition may comprise a very low amount of glycosaminoglycans, *e.g.,* less than 0.1% glycosaminoglycans, less than 0.05% glycosaminoglycans, less than 0.01% glycosaminoglycans, or less than 0.001% glycosaminoglycans. Said ECM composition may comprise glycosaminoglycans, *e.g.,* 0.001 to 0.1% glycosaminoglycans, 0.001 to 0.05% glycosaminoglycans, 0.001 to 0.01% glycosaminoglycans, 0.01 to 0.1% glycosaminoglycans, or 0.01 to 0.05% glycosaminoglycans. Said ECM composition may comprises a very low amount of fibronectin, *e.g.,* less than 0.1% fibronectin, less than 0.01% fibronectin, or less than 0.001% fibronectin; no or an undetectable amount of laminin; and no or an undetectable amount of glycosaminoglycans. Said ECM composition may may comprise fibronectin, *e.g.,* less than 0.1% fibronectin, less than 0.05% fibronectin, less than 0.01% fibronectin, less than 0.001% fibronectin, 0.001 to 0.1% fibronectin, 0.001 to 0.05% fibronectin, 0.001 to 0.01% fibronectin, 0.01 to 0.1% fibronectin, or 0.01 to 0.05% fibronectin, laminin, *e.g.,* less than 0.1% laminin, less than 0.05% laminin, less than 0.01% laminin, less than 0.001% laminin, 0.001 to 0.1% laminin, 0.001 to 0.05% laminin, 0.001 to 0.01% laminin, 0.01 to 0.1% laminin, or 0.01 to 0.05% laminin, glycosaminoglycans, *e.g.,* less than 0.1% glycosaminoglycans, less than 0.05% glycosaminoglycans, less than 0.01% glycosaminoglycans, less than 0.001% glycosaminoglycans, 0.001 to 0.1% glycosaminoglycans, 0.001 to 0.05% glycosaminoglycans, 0.001 to 0.01% glycosaminoglycans, 0.01 to 0.1% glycosaminoglycans, or 0.01 to 0.05% glycosaminoglycans, and, additionally, may comprise no or an undetectable amount of cytokines, growth factors, and/or deoxycholic acid.

Described herein is an ECM composition comprising about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, 60%, 61%, 62%, 63%, 64%, 65%, 66%, 67%, 68%, 69%, 70%,collagen and about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, elastin. Said ECM composition may be obtained from placental tissue, *e.g.,* human placental tissue. Said ECM composition may be obtained from the chorion of a placenta, for example, the chorionic plate, *e.g.,* the chorion, for example, the chorionic plate, from a human placenta. Said ECM composition may comprise a very low amount of fibronectin, *e.g.,* less than 0.1% fibronectin, less than 0.05% fibronectin, less than 0.01% fibronectin, or less than 0.001% fibronectin. Said ECM composition may comprise 0.001 to 0.1% fibronectin, 0.001 to 0.05% fibronectin, 0.001 to 0.01% fibronectin, 0.01 to 0.1% fibronectin, or 0.01 to 0.05% fibronectin. Said Said ECM composition may comprise no or an undetectable amount of laminin. Said ECM composition may comprise a very low amount of laminin, *e.g.,* less than 0.1% laminin, less than 0.05% laminin, less than 0.01% laminin, or less than 0.001% laminin. Said ECM composition may comprise laminin, *e.g.,* 0.001 to 0.1% laminin, 0.001 to 0.05% laminin, 0.001 to 0.01% laminin, 0.01 to 0.1% laminin, or 0.01 to 0.05% laminin. Said ECM composition may comprise no or an undetectable amount of glycosaminoglycans. Said ECM composition may comprise a very low amount of glycosaminoglycans, *e.g.,* less than 0.1% glycosaminoglycans, less than 0.05% glycosaminoglycans, less than 0.01% glycosaminoglycans, or less than 0.001% glycosaminoglycans. Said ECM composition may comprise glycosaminoglycans, *e.g.,* 0.001 to 0.1% glycosaminoglycans, 0.001 to 0.05% glycosaminoglycans, 0.001 to 0.01% glycosaminoglycans, 0.01 to 0.1% glycosaminoglycans, or 0.01 to 0.05% glycosaminoglycans. Said ECM composition may comprise a very low amount of fibronectin, *e.g.,* less than 0.1% fibronectin, less than 0.01% fibronectin, or less than 0.001% fibronectin; no or an undetectable amount of laminin; and no or an undetectable amount of glycosaminoglycans. Said ECM composition may comprise fibronectin, *e.g.,* less than 0.1% fibronectin, less than 0.05% fibronectin, less than 0.01% fibronectin, less than 0.001% fibronectin, 0.001 to 0.1% fibronectin, 0.001 to 0.05% fibronectin, 0.001 to 0.01% fibronectin, 0.01 to 0.1% fibronectin, or 0.01 to 0.05% fibronectin, laminin, *e.g.,* less than 0.1% laminin, less than 0.05% laminin, less than 0.01% laminin, less than 0.001% laminin, 0.001 to 0.1% laminin, 0.001 to 0.05% laminin, 0.001 to 0.01% laminin, 0.01 to 0.1% laminin, or 0.01 to 0.05% laminin, glycosaminoglycans, *e.g.,* less than 0.1% glycosaminoglycans, less than 0.05% glycosaminoglycans, less than 0.01% glycosaminoglycans, less than 0.001% glycosaminoglycans, 0.001 to 0.1% glycosaminoglycans, 0.001 to 0.05% glycosaminoglycans, 0.001 to 0.01% glycosaminoglycans, 0.01 to 0.1% glycosaminoglycans, or 0.01 to 0.05% glycosaminoglycans, and, additionally, may comprise no or an undetectable amount of cytokines, growth factors, and/or deoxycholic acid.

The ECM compositions provided herein can be formulated in multiple ways, and the type of formulation can be selected based on, *e.g.,* the intended use of the ECM composition. Exemplary formulations of the ECM compositions provided herein are detailed in Section 4.1.1. In a specific embodiment, the ECM compositions provided herein are formulated as a flowable matrix, *e.g.,* in a form that can be administered using a syringe. In another specific embodiment, the ECM compositions provided herein are formulated as a particulate, *e.g.,* in powder form. In another specific embodiment, the ECM compositions provided herein are formulated as sheets. An ECM composition described herein may be formulated to comprise one or more components that are non-ECM components. See Section 4.1.1.1.

Described herein are laminates comprising at least one ECM sheet provided herein. Described herein is a laminate comprising two ECM sheets. Described herein is a laminate comprising at least one ECM sheet provided herein and at least one other planar decellularized tissue (*e.g*., decellularized/dehydrated amniotic membrane, either completely decellularized or decellularized so as to retain a fibroblastic cell layer), or with a planar artificial tissue substitute. Laminates can be generated by placing ECM sheets, or one or more ECM sheets and another planar decellularized tissue, in contact with each other in the presence of an adhesive, *e.g.,* a glue *(e.g.,* natural glue, *e.g.,* fibronectin, fibrin; or synthetic glue). Laminates can be generated by heat-drying together two or more ECM sheets, or one or more ECM sheets and one or more planar decellularized tissues.

The ECM compositions provided herein can be seeded with and/or comprise one or more types of cells, *i.e.,* cells can be cultured with and grown upon an ECM composition described herein or dispersed within an ECM composition described herein *(e.g.,* added to an ECM flowable matrix composition). One of skill in the art will appreciate that any cell type known in the art can be seeded with and/or cultured with the ECM compositions provided herein, including both stem cells and non-stem cells. A non-limiting listing of the types of cells that can be seeded on the ECM compositions provided herein is provided in Section 4.1.2.

In another aspect, provided herein are methods of making the ECM compositions according to the claims. See Section 4.2. Methods of making ECM compositions from placenta (*e.g.,* human placenta) that are described herein may comprise the following steps, in order: (i) removing the amnion, chorion, and umbilical cord from a placenta *(e.g.,* from a placenta obtained from a mother immediately after birth, or from a stored placenta); (ii) subjecting the placental tissue to a solution that causes osmotic disruption of cells associated with the placental tissue; (iii) contacting the placenta with a solution comprising a detergent; and (iv) contacting the placenta with a solution comprising a base. Methods of making ECM compositions from placenta may use the chorion of the placenta (*e.g.,* human placenta), wherein said methods comprise the following steps, in order: (i) obtaining the chorion from a placenta (*e.g.,* from a placenta obtained from a mother immediately after birth, or from a stored placenta); (ii) subjecting the chorion to a solution that causes osmotic disruption of cells associated with the chorion; (iii) contacting the chorion with a solution comprising a detergent; and (iv) contacting the chorion with a solution comprising a base. Methods of making ECM compositions from placenta may use the chorion of the placenta (*e.g*., human placenta), wherein said methods comprise the following steps, in order: (i) obtaining the chorion from a placenta (*e.g.*, from a placenta obtained from a mother immediately after birth, or from a stored placenta); (ii) scraping and cleaning the chorion; (iii) subjecting the chorion to a solution that causes osmotic disruption of cells associated with the chorion; (iv) contacting the chorion with a solution comprising a detergent; and (v) grinding and freeze drying. Methods of making ECM compositions from placenta may use the chorion of the placenta (*e.g.*, human placenta), wherein said methods comprise the following steps, in order: (i) obtaining the chorion from a placenta (*e.g*., from a placenta obtained from a mother immediately after birth, or from a stored placenta); (ii) scraping and cleaning the chorion; (iii) subjecting the chorion to a solution that causes osmotic disruption of cells associated with the chorion; (iv) contacting the chorion with a solution comprising with a first, then a second detergent solution, said solutions comprising a detergent and a chelating agent, *e.g.,* EDTA; and (v) freeze drying. The methods of making the ECM compositions described herein use components, *e.g.,* base, detergent, chelating agent, in amounts that result in the generation of ECM compositions having the particular characteristics of those described herein.

The methods of making the ECM compositions described herein use components, *e.g.,* base, detergent, in amounts that result in the generation of ECM compositions having the particular characteristics of those described herein.

In a specific embodiment, provided herein is a method of making an ECM composition, the ECM composition comprising 35-55% collagen by dry weight and 1 0-30% elastin by dry weight, said method comprising (i) removing the amnion, chorion, and umbilical cord from a placenta *(e.g.,* from a placenta obtained from a mother immediately after birth, or from a stored placenta); comprising (ii) placing the remaining placental tissue in a solution comprising 1 M NaCl and homogenizing the placental tissue; (iii) contacting the placental tissue with a solution comprising 2% sodium deoxycholate; (iv) washing the placental tissue with water; (v) contacting the placental tissue with a solution comprising a 1 M NaOH; (vi) adding an acid solution, *e.g.,* hydrochloric acid (HCl), to the solution comprising placental tissue to bring it to or close to a neutral pH *(e.g.,* pH 6.0-8.0); and (vii) separating the placental tissue from the liquid portion of the solution (*e.g*., by centrifugation) and collecting the placental tissue, thereby making an ECM composition. The ECM composition generated according to the method generally is in the form of a paste (ECM paste), which can be frozen and stored after collection for later use, or which can be used directly after collection to manufacture an ECM formulation described herein, *e.g.,* in the formulation of an ECM sheet, an ECM particulate formulation, or an ECM flowable matrix.

Described herein is a method of making an ECM composition, said method comprising (i) obtaining the chorion from a placenta (*e.g*., from a placenta obtained from a mother immediately after birth, or from a stored placenta); (ii) placing the chorion tissue in a solution that causes osmotic disruption of cells associated with the chorion tissue, *e.g.,* sodium chloride (NaCl, *e.g.,* 1 M NaCl) and homogenizing the chorion tissue; (iii) contacting the chorion tissue with a solution comprising a detergent, *e.g.,* sodium deoxycholate *(e.g.,* 2% sodium deoxycholate); (iv) washing the chorion tissue, *e.g.,* with water; (v) contacting the chorion tissue with a solution comprising a base, *e.g.,* sodium hydroxide (NaOH, *e.g.,* 1 M NaOH); (vi) adding an acid solution, *e.g.,* hydrochloric acid (HCl), to the solution comprising chorion tissue to bring it to or close to a neutral pH *(e.g.,* pH 6.0-8.0); and (vii) separating the chorion tissue from the liquid portion of the solution (*e.g*., by centrifugation) and collecting the chorion tissue, thereby making an ECM composition. The ECM composition generated according to the method generally is in the form of a paste (ECM paste), which can be frozen and stored after collection for later use, or which can be used directly after collection to manufacture an ECM formulation described herein, *e.g.,* in the formulation of an ECM sheet, an ECM particulate formulation, or an ECM flowable matrix.

In another specific embodiment, provided herein is a method of making an ECM composition, comprising 40-70% collagen by dry weight and 15-25% elastin by dry weight, said method comprising (i) obtaining the chorion, for example, chorionic plate, from a placenta (*e.g.*, from a placenta obtained from a mother immediately after birth, or from a stored placenta); (ii) scraping and cleaning the chorion; (iii) placing the chorion tissue in a solution comprising 0.5 M NaCl; (iv) contacting the chorion tissue with a solution comprising 2% deoxycholic acid or sodium deoxycholate and rinsing with water; and (v) grinding and freeze drying. The ECM composition, generally a paste (ECM paste), can be formulated, for example, milled and formulated, into a variety of shapes and forms, *e.g.,* an ECM sheet, an ECM particulate formulation, or an ECM flowable matrix.

In yet another specific embodiment, provided herein is a method of making an ECM composition, comprising 40-70% collagen by dry weight and 15-25% elastin by fry weight, said method comprising (i) obtaining the chorion, for example, chorionic plate, from a placenta (*e.g.*, from a placenta obtained from a mother immediately after birth, or from a stored placenta); (ii) scraping and cleaning the chorion; (iii) placing the chorion tissue in a comprising solution comprising 1.0 M NaCl; (iv) contacting the chorion tissue with a first detergent solution comprising 0.05%-0.1% deoxycholic acid or sodium deoxycholate and 3mM-5mM EDTA followed by water rinsing (v) contacting the chorion tissue with a second detergent solution comprising 0.05%-0.1% deoxycholic acid or sodium deoxycholate) and 3mM-5mM EDTA), followed by water rinsing (vi) freeze drying to yield a decellularized, freeze dried whole chorion which can be formulated, for example, milled and resuspended in solution (*e.g.*, water or phosphate-buffered saline) to form a decellularized ECM paste, and formulated, into a variety of shapes and forms, *e.g.,* an ECM sheet, an ECM particulate formulation, or an ECM flowable matrix.

Described herein is a method of generating an ECM sheet, said method comprising (i) preparing an ECM paste according to the methods described herein; (ii) suspending the ECM paste in, *e.g.,* water, and adding the suspended ECM solution to a suitable substrate for formation of a sheet, *e.g.,* adding the ECM to a mold; (iii) freezing the ECM; (iv) lyophilizing the frozen ECM; (v) removing the lyophilized ECM from the substrate and soaking it in water; and (vi) drying the ECM, *e.g.,* using a vacuum dryer.

Described herein is a method of generating an ECM particulate, said method comprising (i) preparing an ECM paste according to the methods described herein; (ii) suspending the ECM paste in, *e.g.,* water; (iii) freezing the ECM; (iv) lyophilizing the frozen ECM; and (v) milling the lyophilized ECM.

Described herein is a method of generating an ECM flowable matrix, said method comprising (i) preparing an ECM paste according to the methods described herein; (ii) suspending the ECM paste in, *e.g.,* water; (iii) freezing the ECM; (iv) lyophilizing the frozen ECM; and (v) micronizing the lyophilized ECM. Upon resuspension of the micronized ECM in, *e.g.,* saline, an ECM flowable matrix is generated.

In another aspect, provided herein are uses of the ECM compositions according to the claims. See Section 4.3. In certain embodiments, the ECM compositions provided herein are used in a method of treatment. See Section 4.3.1. In certain embodiments, the ECM compositions provided herein are used for cosmetic purposes for treating a cosmetic defect in a subject. See Section 4.3.2.

The ECM compositions provided herein may be used in wound treatment and/or management. See Section 4.3.1. The ECM may be compositions provided herein may be used to fill a wound, that is, as a wound filler. The ECM compositions provided herein may be used to dress (*i.e.,* cover) a wound, *e.g.,* a wound caused by a burn.

The ECM compositions provided herein may be used in the treatment and/or management of a dental condition, *e.g.,* to repair a dental defect. See Section 4.3.2.

In a specific embodiment, the ECM compositions provided herein are used in the treatment and/or management of oral lesions. See Section 4.3.3.

The ECM compositions provided herein may be used to seal, fill, and/or otherwise treat a void within the body of a subject. See Section 4.3.4.

The ECM compositions provided herein may be used for tissue bulking in a subject. See Section 4.3.5.

In another specific embodiment, the ECM compositions provided herein are used for treatment of urinary incontinence in a subject. See Section 4.3.6.

In another specific embodiment, the ECM compositions provided herein are used for treatment of vesicoureteral reflux in a subject. See Section 4.3.7.

The ECM compositions provided herein may be used for treatment of gastroesophageal reflux disease in a subject. See Section 4.3.8.

In another specific embodiment, the ECM compositions provided herein are used for treatment of a disease, disorder, or other abnormality that affects one or both vocal cords and/or the larynx in a subject. See Section 4.3.9.

In another specific embodiment, the ECM compositions provided herein are used for management or treatment of glottic incompetence in a subject. See Section 4.3.10.

The ECM compositions provided herein may be used for bioengineering of tissue or organs. See Section 4.3.11.

The ECM compositions provided herein may be for cosmetic purposes, *e.g.,* to augment skin of a subject for a cosmetic purpose *(e.g.,* to make the subject appear more youthful). See Section 4.3.12.

Described herein, but not explicitly claimed, are kits comprising the ECM compositions provided herein. The kits described herein typically comprise an ECM composition described herein in a package convenient for distribution to a practitioner of skill in the art. See Section 5.

### 3.1 BRIEF DESCRIPTION OF THE DRAWINGS

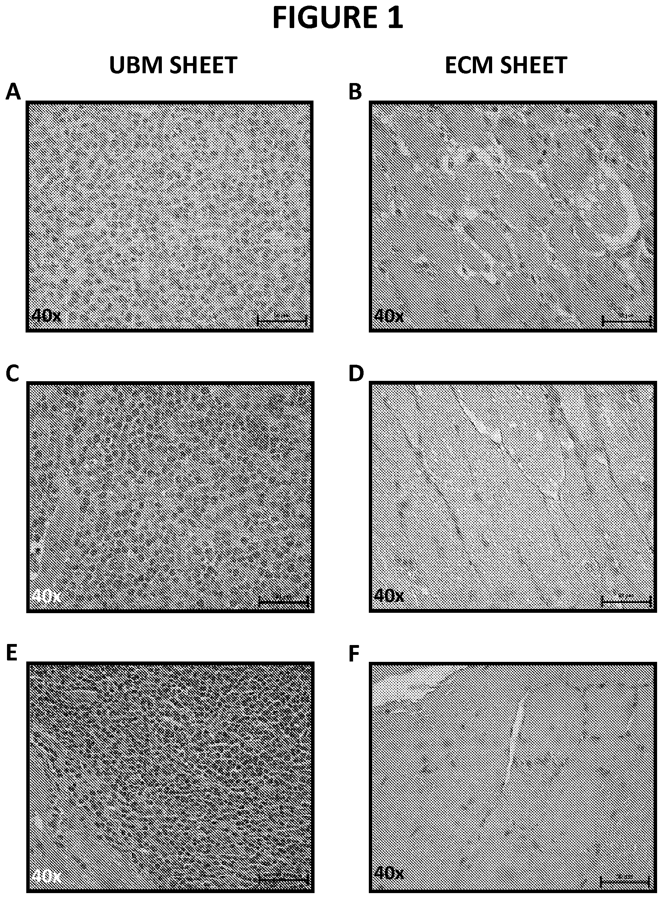

Figure 1A to 1F: Tissue reactivity to ECM Sheet prepared according to the methods described herein as compared to tissue reactivity to porcine urinary bladder matrix following implantation in albino New Zealand White Rabbits. At week 1 postimplantation, the tissue adjacent to the urinary bladder matrix (UBM) sheet showed distinct signs of granulation and inflammatory response (Fig. 1A), while the ECM sheet showed muscle tissue interspersed with slight infiltration of granulocytes (Fig. 1B). At weeks 2 and 4, granulation was still evident adjacent to the UBM sheet (Figs. 1C and 1E), while tissue adjacent to the ECM sheet showed virtually no granulation and appeared to be normal (Figs. 1D and 1F).

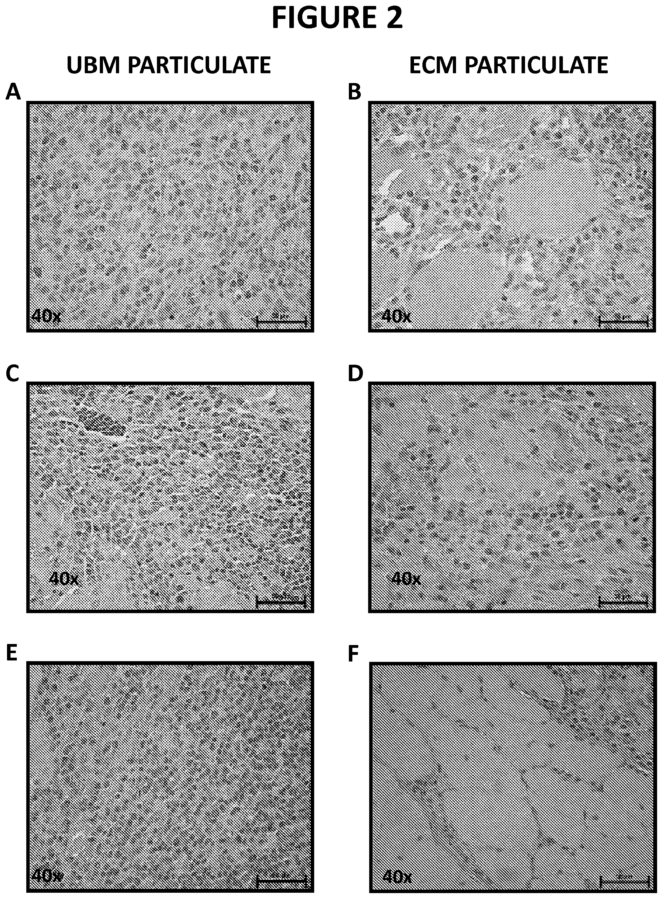

Figure 2A to 2F: Tissue reactivity to ECM particulate prepared according to the methods described herein as compared to tissue reactivity to MATRISTEM MICROMATRIX^{®} following implantation in albino New Zealand White Rabbits. At week 1 postimplantation, the ECM particulate showed some granulation indicating inflammation (FIG. 2B), but significantly less than the UBM particulate (FIG. 2A), while at weeks 2 and 4, the ECM particulate showed a significant reduction of granulation (FIGS. 2D and 2F, respectively) as compared to the UBM particulate, which still showed substantial inflammation at weeks 2 and 4 (FIGS. 2C and 2E, respectively).

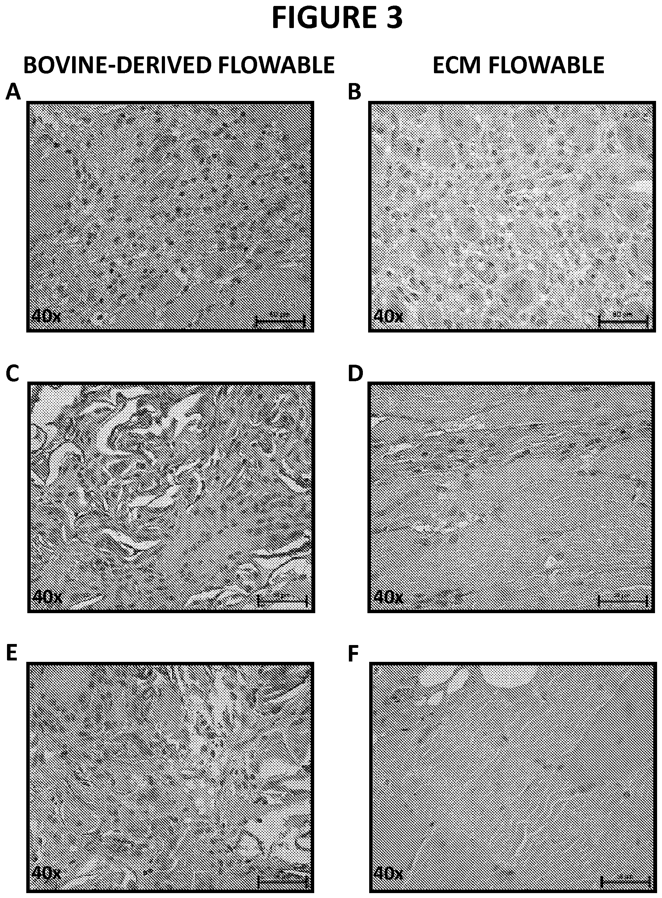

Figure 3A to 3F: Tissue reactivity to ECM flowable matrix prepared according to the methods described herein as compared to tissue reactivity to a bovine derived wound matrix product (INTEGRA^{™} Flowable) following implantation in albino New Zealand White Rabbits. The bovine derived wound matrix product (INTEGRA^{™} Flowable) showed granulation at week 1 (FIG. 3A), followed by scarring at weeks 2 and 4 (lighter areas in FIGS. 3C and 3E), whereas the ECM flowable matrix showed an inflammatory response substantially only in the first week (FIG. 3B), followed by near-complete healing at weeks 2 and 4 (FIGS. 3D and 3F, respectively).

### 4. DETAILED DESCRIPTION

Provided herein are extracellular matrix (ECM) compositions (see Section 4.1) and methods of making the same (see Section 4.2). Also provided herein are uses of the ECM compositions provided herein (see Section 4.3) and kits comprising the ECM compositions provided herein (see Section 5).

### 4.1. Extracellular Matrix Compositions

In one aspect, provided herein are extracellular matrix (ECM) compositions prepared using placental tissue, *e*.*g*., human placental tissue. The ECM compositions described herein comprise ECM components, *e*.*g*., collagen and elastin, in amounts distinct from those in ECM compositions known in the art.

The ECM compositions provided herein may comprise about 30% to about 60% collagen and about 10% to about 35% elastin. As used herein, the term "about" refers to an amount that is plus or minus 10 percent of a specified number. In addition, such ECM compositions provided herein may comprise (i) very low amounts of fibronectin (*e*.*g*., less than 0.1% fibronectin), *e*.*g*., as measured by ELISA; (ii) no or an undetectable amount of laminin, *e.g.,* as measured by ELISA; and/or no or an undetectable amount of glycosaminoglycans, *e.g*., as measured by ELISA.

The ECM compositions provided herein may comprise about 30% to about 72% collagen and about 10% to about 35% elastin. In addition, such ECM compositions can comprise (i) fibronectin *(e.g.,* less than 0.1% fibronectin), *e.g.,* as measured by ELISA; (ii) laminin *(e.g.,* less than 0.1% laminin), *e.g.,* as measured by ELISA; glycosaminoglycans, (*e*.*g*., less than 0.1% glycosaminoglycans) *e*.*g*., as measured by ELISA; (iii) no or an undetectable amount of cytokines; (iv) no or an undetectable amount of growth factors; and/or (v) no or an undetectable amount of deoxycholic acid.

The ECM compositions provided herein comprise characteristics that make them well-suited for therapeutic/medical use. Specifically, the ECM compositions described herein are sterile, acellular *(e.g.,* ≥99% cell-free) and/or are free of cellular debris *(e.g.,* ≥99% free of cellular debris). In particular embodiments, the ECM compositions provided herein and comprise no cytokines or an undectable amount of cytokines, as measured by, *e.g.,* ELISA. The ECM compositions provided herein further may be devoid of reagent residuals, *i*.*e*., the final ECM compositions comprise undetectable amounts of reagents used in the manufacture of the compositions. The ECM compositions provided herein may comprise minimal amounts of nucleic acid (-41-171 ng/mg of dry product) and endotoxin (<0.25 EU).

Another advantageous characteristic of the ECM compositions provided herein is their ability to absorb water. The ECM compositions provided herein may absorb between 150%-225% their weight in water.

Described herein is an ECM composition comprising 34-53% collagen and 13-29% elastin. Said ECM composition may be obtained from placental tissue, *e*.*g*., human placental tissue. Said ECM composition may be obtained from the chorion of a placenta, *e.g.,* the chorion from a human placenta. Said ECM composition may comprise a very low amount of fibronectin, *e*.*g*., less than 0.1% fibronectin, less than 0.01% fibronectin, or less than 0.001% fibronectin, Said ECM composition may comprise no or an undetectable amount of laminin. Said ECM composition may comprise no or an undetectable amount of glycosaminoglycans. Said ECM composition may comprise a very low amount of fibronectin, *e.g.,* less than 0.1% fibronectin, less than 0.01% fibronectin, or less than 0.001% fibronectin; no or an undetectable amount of laminin; and no or an undetectable amount of glycosaminoglycans.

In another specific embodiment provided herein is an ECM composition comprising about 35-55% collagen and about 10-30% elastin. Said ECM composition may be obtained from placental tissue, *e*.*g*., human placental tissue. Said ECM composition may be obtained from the chorion of a placenta, *e.g.,* the chorion from a human placenta. In another specific embodiment, said ECM composition comprises a very low amount of fibronectin, *e*.*g*., less than 0.1% fibronectin, less than 0.01% fibronectin, or less than 0.001% fibronectin. In another specific embodiment, said ECM composition comprises an undetectable amount of laminin. In another specific embodiment, said ECM composition comprises an undetectable amount of glycosaminoglycans. In another specific embodiment, said ECM composition comprises a very low amount of fibronectin, *e*.*g*., less than 0.1% fibronectin, less than 0.01% fibronectin, or less than 0.001% fibronectin; an undetectable amount of laminin; and an undetectable amount of glycosaminoglycans.

Described herein is an ECM composition comprising about 35-72% collagen and about 15-25% elastin. In another specific embodiment provided herein is an ECM composition comprising about 40-70% collagen and about 15-25% elastin. Said composition may comprise 40-70% collagen and 15-22% elastin. Said composition may comprise 43-68% collagen and 18-21% elastin. In certain embodiments, said ECM composition comprises less than 0.1% fibronectin, less than 0.05% fibronectin, less than 0.01% fibronectin, less than 0.001% fibronectin, 0.001 to 0.1% fibronectin, 0.001 to 0.05% fibronectin, 0.001 to 0.01% fibronectin, 0.01 to 0.1% fibronectin, or 0.01 to 0.05% fibronectin. In another specific embodiment, said ECM composition comprises less than 0.1% laminin, less than 0.05% laminin, less than 0.01% laminin, less than 0.001% laminin, 0.001 to 0.1% laminin, 0.001 to 0.05% laminin, 0.001 to 0.01% laminin, 0.01 to 0.1% laminin, or 0.01 to 0.05% laminin. In another specific embodiment, said ECM composition comprises less than 0.1% glycosaminoglycans, less than 0.05% glycosaminoglycans, less than 0.01% glycosaminoglycans, less than 0.001% glycosaminoglycans, 0.001 to 0.1% glycosaminoglycans, 0.001 to 0.05% glycosaminoglycans, 0.001 to 0.01% glycosaminoglycans, 0.01 to 0.1% glycosaminoglycans, or 0.01 to 0.05% glycosaminoglycans. In another specific embodiment, said ECM composition comprises an undetectable amount of cytokines, growth factors, and/or deoxycholic acid. In certain embodiments, said ECM composition comprises less than 0.1% fibronectin, less than 0.05% fibronectin, less than 0.01% fibronectin, less than 0.001% fibronectin, 0.001 to 0.1% fibronectin, 0.001 to 0.05% fibronectin, 0.001 to 0.01% fibronectin, 0.01 to 0.1% fibronectin, or 0.01 to 0.05% fibronectin, less than 0.1% laminin, less than 0.05% laminin, less than 0.01% laminin, less than 0.001% laminin, 0.001 to 0.1% laminin, 0.001 to 0.05% laminin, 0.001 to 0.01% laminin, 0.01 to 0.1% laminin, or 0.01 to 0.05% laminin, less than 0.1% glycosaminoglycans, less than 0.05% glycosaminoglycans, less than 0.01% glycosaminoglycans, less than 0.001% glycosaminoglycans, 0.001 to 0.1% glycosaminoglycans, 0.001 to 0.05% glycosaminoglycans, 0.001 to 0.01% glycosaminoglycans, 0.01 to 0.1% glycosaminoglycans, or 0.01 to 0.05% glycosaminoglycans, and, additionally, comprises an undetectable amount of cytokines, growth factors, and/or deoxycholic acid. Said ECM composition may be obtained from placental tissue, *e*.*g*., human placental tissue. Said ECM composition is obtained from the chorion of a placenta, for example the chorionic plate of a placenta, *e*.*g*., the chorion, for example the chorionic plate, from a human placenta.

Described herein is an ECM composition comprising about 50-60% collagen and about 10-20% elastin. Said ECM composition may be obtained from placental tissue, *e*.*g*., human placental tissue. Said ECM composition may be obtained from the chorion of a placenta, *e*.*g*., the chorion from a human placenta. Said ECM composition may comprise a very low amount of fibronectin, *e*.*g*., less than 0.1% fibronectin, less than 0.01% fibronectin, or less than 0.001% fibronectin. Said ECM composition may comprise no or an undetectable amount of laminin. Said ECM composition may comprise no or an undetectable amount of glycosaminoglycans. Said ECM composition may comprise a very low amount of fibronectin, *e*.*g*., less than 0.1% fibronectin, less than 0.01% fibronectin, or less than 0.001% fibronectin; no or an undetectable amount of laminin; and no or an undetectable amount of glycosaminoglycans.

Descrbied herein is an ECM composition comprising about 45-55% collagen and about 15-25% elastin. Said ECM composition may be obtained from placental tissue, *e*.*g*., human placental tissue. Said ECM composition may be obtained from the chorion of a placenta, *e*.*g*., the chorion from a human placenta. Said ECM composition may comprise a very low amount of fibronectin, *e*.*g*., less than 0.1% fibronectin, less than 0.01% fibronectin, or less than 0.001% fibronectin. Said ECM composition may comprise no or an undetectable amount of laminin. Said ECM composition may comprise no or an undetectable amount of glycosaminoglycans. Said composition may comprise a very low amount of fibronectin, *e*.*g*., less than 0.1% fibronectin, less than 0.01% fibronectin, or less than 0.001% fibronectin; no or an undetectable amount of laminin; and no or an undetectable amount of glycosaminoglycans.

Described herein is an ECM composition comprising about 40-50% collagen and about 20-30% elastin. Said ECM composition may be obtained from placental tissue, *e*.*g*., human placental tissue. Said ECM composition may be obtained from the chorion of a placenta, *e.g.,* the chorion from a human placenta. Said ECM composition may comprise a very low amount of fibronectin, *e*.*g*., less than 0.1% fibronectin, less than 0.01% fibronectin, or less than 0.001% fibronectin. Said ECM composition may comprise no or an undetectable amount of laminin. Said ECM composition may comprise no or an undetectable amount of glycosaminoglycans. Said ECM composition may comprise a very low amount of fibronectin, *e*.*g*., less than 0.1% fibronectin, less than 0.01% fibronectin, or less than 0.001% fibronectin; no or an undetectable amount of laminin; and no or an undetectable amount of glycosaminoglycans.

Described herein is an ECM composition comprising about 30-40% collagen and about 25-35% elastin. Said ECM composition may be obtained from placental tissue, *e*.*g*., human placental tissue. Said ECM composition may be obtained from the chorion of a placenta, *e.g.,* the chorion from a human placenta. Said ECM composition may comprise a very low amount of fibronectin, *e*.*g*., less than 0.1% fibronectin, less than 0.01% fibronectin, or less than 0.001% fibronectin. Said ECM composition may comprise no or an undetectable amount of laminin. Said ECM composition may comprise no or an undetectable amount of glycosaminoglycans. Said ECM composition may comprise a very low amount of fibronectin, *e*.*g*., less than 0.1% fibronectin, less than 0.01% fibronectin, or less than 0.001% fibronectin; no or an undetectable amount of laminin; and no or an undetectable amount of glycosaminoglycans.

Described herein is an ECM composition comprising about 34-43% collagen and about 16-24% elastin. Said ECM composition may be obtained from placental tissue, *e*.*g*., human placental tissue. Said ECM composition may be obtained from the chorion of a placenta, *e.g.,* the chorion from a human placenta. Said ECM composition may comprise a very low amount of fibronectin, *e*.*g*., less than 0.1% fibronectin, less than 0.01% fibronectin, or less than 0.001% fibronectin. Said ECM composition may comprise no or an undetectable amount of laminin. Said ECM composition may comprise no or an undetectable amount of glycosaminoglycans. Said ECM composition may comprise a very low amount of fibronectin, *e*.*g*., less than 0.1% fibronectin, less than 0.01% fibronectin, or less than 0.001% fibronectin; no or an undetectable amount of laminin; and no or an undetectable amount of glycosaminoglycans.

Described herein is an ECM composition comprising about 37-42% collagen and about 16-24% elastin. Said ECM composition may be obtained from placental tissue, *e*.*g*., human placental tissue. Said ECM composition is obtained from the chorion of a placenta, *e.g.,* the chorion from a human placenta. Said ECM composition may comprise a very low amount of fibronectin, *e*.*g*., less than 0.1% fibronectin, less than 0.01% fibronectin, or less than 0.001% fibronectin. Said ECM composition may comprise no or an undetectable amount of laminin. Said ECM composition may comprise no or an undetectable amount of glycosaminoglycans. Said ECM composition may comprise a very low amount of fibronectin, *e*.*g*., less than 0.1% fibronectin, less than 0.01% fibronectin, or less than 0.001% fibronectin; no or an undetectable amount of laminin; and no or an undetectable amount of glycosaminoglycans.

Described herein is an ECM composition comprising about 35-40%, 37-42%, 40-70%, 40-60%, 40-50%, 40-45%, 40-65%, 43-68%, 45-50%, 50-55%, or 55-60% collagen and about 10-30%, 10-20%, 10-15%, 15-25%, 15-22%, 15-20%, 16-24%, 17-24%, 18-21%, 18-20%, 20-24%, 20-30%, 20-25%, or about 25-30%, elastin. Said ECM composition may be obtained from placental tissue, *e*.*g*., human placental tissue. Said ECM composition may be obtained from the chorion of a placenta, for example the chorionic plate, *e.g.,* the chorion, for example, the chorionic plate, from a human placenta, Said ECM composition may comprise a very low amount of fibronectin, *e*.*g*., less than 0.1% fibronectin, less than 0.05% fibronectin, less than 0.01% fibronectin, or less than 0.001% fibronectin. Said ECM composition may may comprise 0.001 to 0.1% fibronectin, 0.001 to 0.05% fibronectin, 0.001 to 0.01% fibronectin, 0.01 to 0.1% fibronectin, or 0.01 to 0.05% fibronectin. Said ECM composition may comprise no or an undetectable amount of laminin. Said ECM composition may comprise a very low amount of laminin, *e.g.,* less than 0.1% laminin, less than 0.05% laminin, less than 0.01% laminin, or less than 0.001% laminin. Said ECM composition may comprise laminin, *e*.*g*., 0.001 to 0.1% laminin, 0.001 to 0.05% laminin, 0.001 to 0.01% laminin, 0.01 to 0.1% laminin, or 0.01 to 0.05% laminin. Said ECM composition may comprise no or an undetectable amount of glycosaminoglycans. Said ECM composition may comprise a very low amount of glycosaminoglycans, *e*.*g*., less than 0.1% glycosaminoglycans, less than 0.05% glycosaminoglycans, less than 0.01% glycosaminoglycans, or less than 0.001% glycosaminoglycans. Said ECM composition may comprise glycosaminoglycans, *e*.*g*., 0.001 to 0.1% glycosaminoglycans, 0.001 to 0.05% glycosaminoglycans, 0.001 to 0.01% glycosaminoglycans, 0.01 to 0.1% glycosaminoglycans, or 0.01 to 0.05% glycosaminoglycans. Said ECM composition may comprise a very low amount of fibronectin, *e*.*g*., less than 0.1% fibronectin, less than 0.01% fibronectin, or less than 0.001% fibronectin; no or an undetectable amount of laminin; and no or an undetectable amount of glycosaminoglycans. Said ECM composition may comprise fibronectin, *e.g.,* less than 0.1% fibronectin, less than 0.05% fibronectin, less than 0.01% fibronectin, less than 0.001% fibronectin, 0.001 to 0.1% fibronectin, 0.001 to 0.05% fibronectin, 0.001 to 0.01% fibronectin, 0.01 to 0.1% fibronectin, or 0.01 to 0.05% fibronectin, laminin, *e*.*g*., less than 0.1% laminin, less than 0.05% laminin, less than 0.01% laminin, less than 0.001% laminin, 0.001 to 0.1% laminin, 0.001 to 0.05% laminin, 0.001 to 0.01% laminin, 0.01 to 0.1% laminin, or 0.01 to 0.05% laminin, glycosaminoglycans, *e.g.,* less than 0.1% glycosaminoglycans, less than 0.05% glycosaminoglycans, less than 0.01% glycosaminoglycans, less than 0.001% glycosaminoglycans, 0.001 to 0.1% glycosaminoglycans, 0.001 to 0.05% glycosaminoglycans, 0.001 to 0.01% glycosaminoglycans, 0.01 to 0.1% glycosaminoglycans, or 0.01 to 0.05% glycosaminoglycans, and, additionally, comprise no or an undetectable amount of cytokines, growth factors, and/or deoxycholic acid.

Described herein is an ECM composition comprising about 30%, about 35%, about 40%, about 45%, about 50%, about 55%, about 60%, about 65%, or about 70% collagen and about 10%, about 15%, about 18%, about 20%, about 25%, or about 30% elastin. Said ECM composition may be obtained from placental tissue, *e*.*g*., human placental tissue. Said ECM composition may be obtained from the chorion of a placenta, for example, the chorionic plate, *e.g.,* the chorion, for example, the chorionic plate, from a human placenta. Said ECM composition may comprise a very low amount of fibronectin, *e*.*g*., less than 0.1% fibronectin, less than 0.05% fibronectin, less than 0.01% fibronectin, or less than 0.001% fibronectin. Said ECM composition may comprise 0.001 to 0.1% fibronectin, 0.001 to 0.05% fibronectin, 0.001 to 0.01% fibronectin, 0.01 to 0.1% fibronectin, or 0.01 to 0.05% fibronectin. Said ECM composition may comprise no or an undetectable amount of laminin. Said ECM composition may comprise a very low amount of laminin, *e*.*g*., less than 0.1% laminin, less than 0.05% laminin, less than 0.01% laminin, or less than 0.001% laminin. Said ECM composition may comprise laminin, *e*.*g*., 0.001 to 0.1% laminin, 0.001 to 0.05% laminin, 0.001 to 0.01% laminin, 0.01 to 0.1% laminin, or 0.01 to 0.05% laminin. Said ECM composition may comprise no or an undetectable amount of glycosaminoglycans. Said ECM composition may comprise a very low amount of glycosaminoglycans, *e*.*g*., less than 0.1% glycosaminoglycans, less than 0.05% glycosaminoglycans, less than 0.01% glycosaminoglycans, or less than 0.001% glycosaminoglycans. Said ECM composition may comprise glycosaminoglycans, *e*.*g*., 0.001 to 0.1% glycosaminoglycans, 0.001 to 0.05% glycosaminoglycans, 0.001 to 0.01% glycosaminoglycans, 0.01 to 0.1% glycosaminoglycans, or 0.01 to 0.05% glycosaminoglycans. Said ECM composition may comprise a very low amount of fibronectin, *e*.*g*., less than 0.1% fibronectin, less than 0.01% fibronectin, or less than 0.001% fibronectin; no or an undetectable amount of laminin; and no or an undetectable amount of glycosaminoglycans. Said ECM composition may comprise fibronectin, *e*.*g*., less than 0.1% fibronectin, less than 0.05% fibronectin, less than 0.01% fibronectin, less than 0.001% fibronectin, 0.001 to 0.1% fibronectin, 0.001 to 0.05% fibronectin, 0.001 to 0.01% fibronectin, 0.01 to 0.1% fibronectin, or 0.01 to 0.05% fibronectin, laminin, *e*.*g*., less than 0.1% laminin, less than 0.05% laminin, less than 0.01% laminin, less than 0.001% laminin, 0.001 to 0.1% laminin, 0.001 to 0.05% laminin, 0.001 to 0.01% laminin, 0.01 to 0.1% laminin, or 0.01 to 0.05% laminin, glycosaminoglycans, *e.g.,* less than 0.1% glycosaminoglycans, less than 0.05% glycosaminoglycans, less than 0.01% glycosaminoglycans, less than 0.001% glycosaminoglycans, 0.001 to 0.1% glycosaminoglycans, 0.001 to 0.05% glycosaminoglycans, 0.001 to 0.01% glycosaminoglycans, 0.01 to 0.1% glycosaminoglycans, or 0.01 to 0.05% glycosaminoglycans, and, additionally, may comprise no or an undetectable amount of cytokines, growth factors, and/or deoxycholic acid.

Described herein is an ECM composition comprising about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, 60%, 61%, 62%, 63%, 64%, 65%, 66%, 67%, 68%, 69%, 70%, or about 71% collagen and about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, or about 29%, /about 30% elastin. Said ECM composition may be obtained from placental tissue, *e*.*g*., human placental tissue. Said ECM composition may be obtained from the chorion of a placenta, for example, the chorionic plate, *e.g.,* the chorion, for example, the chorionic plate, from a human placenta. Said ECM composition may comprise a very low amount of fibronectin, *e*.*g*., less than 0.1% fibronectin, less than 0.05% fibronectin, less than 0.01% fibronectin, or less than 0.001% fibronectin. Said ECM composition may comprise 0.001 to 0.1% fibronectin, 0.001 to 0.05% fibronectin, 0.001 to 0.01% fibronectin, 0.01 to 0.1% fibronectin, or 0.01 to 0.05% fibronectin. Said ECM composition may comprise no or an undetectable amount of laminin. Said ECM composition may comprise a very low amount of laminin, *e*.*g*., less than 0.1% laminin, less than 0.05% laminin, less than 0.01% laminin, or less than 0.001% laminin. Said ECM composition may comprise laminin, *e*.*g*., 0.001 to 0.1% laminin, 0.001 to 0.05% laminin, 0.001 to 0.01% laminin, 0.01 to 0.1% laminin, or 0.01 to 0.05% laminin. Said ECM composition may comprise no or an undetectable amount of glycosaminoglycans. Said ECM composition may comprise a very low amount of glycosaminoglycans, *e*.*g*., less than 0.1% glycosaminoglycans, less than 0.05% glycosaminoglycans, less than 0.01% glycosaminoglycans, or less than 0.001% glycosaminoglycans. Said ECM composition may comprise glycosaminoglycans, *e*.*g*., 0.001 to 0.1% glycosaminoglycans, 0.001 to 0.05% glycosaminoglycans, 0.001 to 0.01% glycosaminoglycans, 0.01 to 0.1% glycosaminoglycans, or 0.01 to 0.05% glycosaminoglycans. Said ECM composition may comprise a very low amount of fibronectin, *e*.*g*., less than 0.1% fibronectin, less than 0.01% fibronectin, or less than 0.001% fibronectin; no or an undetectable amount of laminin; and no or an undetectable amount of glycosaminoglycans. Said ECM composition may comprise fibronectin, *e*.*g*., less than 0.1% fibronectin, less than 0.05% fibronectin, less than 0.01% fibronectin, less than 0.001% fibronectin, 0.001 to 0.1% fibronectin, 0.001 to 0.05% fibronectin, 0.001 to 0.01% fibronectin, 0.01 to 0.1% fibronectin, or 0.01 to 0.05% fibronectin, laminin, e.g., less than 0.1% laminin, less than 0.05% laminin, less than 0.01% laminin, less than 0.001% laminin, 0.001 to 0.1% laminin, 0.001 to 0.05% laminin, 0.001 to 0.01% laminin, 0.01 to 0.1% laminin, or 0.01 to 0.05% laminin, glycosaminoglycans, *e.g.,* less than 0.1% glycosaminoglycans, less than 0.05% glycosaminoglycans, less than 0.01% glycosaminoglycans, less than 0.001% glycosaminoglycans, 0.001 to 0.1% glycosaminoglycans, 0.001 to 0.05% glycosaminoglycans, 0.001 to 0.01% glycosaminoglycans, 0.01 to 0.1% glycosaminoglycans, or 0.01 to 0.05% glycosaminoglycans, and, additionally, may comprise no or an undetectable amount of cytokines, growth factors, and/or deoxycholic acid.

The collagen in the ECM compositions described herein may comprise or consist of telopeptide collagen. The collagen in the ECM compositions described herein may comprise or consist of atelopeptide collagen. It may consist of telopeptide collagen and atelopeptide collagen.

The primary type of collagen in the ECM compositions provided herein is type I collagen. The collagen in the ECM compositions provided herein may comprise at least 60%, at least 65%, at least 70%, at least 75%, at least 80%, or greater than 80% type I collagen by dry weight. The collagen in the ECM compositions provided herein may comprise between 50% and 70% type I collagen, between 60% and 80% type I collagen, or between 70% and 90% type I collagen by dry weight. The collagen in the ECM compositions provided herein may comprise between 60% and 80% type I collagen.

The collagen in the ECM compositions provided herein may comprise a mixture of collagen types, *e.g*. may comprise type I collagen as well as type III collagen and/or type IV collagen.

The collagen in the ECM compositions provided herein may comprise a substantial amount of type I collagen *(e.g.,* 60%-80% type I collagen) while also comprising type III collagen. For example, in addition to type I collagen, the collagen in the ECM compositions provided herein can comprise between 1% and 5% type III collagen, between 5% and 10% type III collagen, or between 1% and 10% type III collagen by dry weight; or the collagen in the ECM compositions provided herein can comprise about 1%, about 2%, about 3%, about 4%, about 5%, about 6%, about 7%, about 8%, about 9%, or about 10% type III collagen by dry weight.

The collagen in the ECM compositions provided herein may comprise a substantial amount of type I collagen *(e.g.,* 60%-80% type I collagen) while also comprising type IV collagen. For example, in addition to type I collagen, the collagen in the ECM compositions provided herein can comprise between 1% and 5% type IV collagen, between 5% and 10% type IV collagen, or between 1% and 10% type IV collagen by dry weight; or the collagen in the ECM compositions provided herein can comprise about 1%, about 2%, about 3%, about 4%, about 5%, about 6%, about 7%, about 8%, about 9%, or about 10% type IV collagen by dry weight.

The collagen in the ECM compositions provided herein may comprise a substantial amount of type I collagen *(e.g.,* 60%-80% type I collagen) while also comprising type III collagen and type IV collagen. For example, in addition to type I collagen, the collagen in the ECM compositions provided herein can comprise (i) between 1% and 5% type III collagen, between 5% and 10% type III collagen, or between 1% and 10% type III collagen by dry weight; or the collagen in the ECM compositions provided herein can comprise about 1%, about 2%, about 3%, about 4%, about 5%, about 6%, about 7%, about 8%, about 9%, or about 10% type III collagen by dry weight; and (ii) between 1% and 5% type IV collagen, between 5% and 10% type IV collagen, or between 1% and 10% type IV collagen by dry weight; or the collagen in the ECM compositions provided herein can comprise about 1%, about 2%, about 3%, about 4%, about 5%, about 6%, about 7%, about 8%, about 9%, or about 10% type IV collagen by dry weight.

The collagen in the ECM compositions provided herein may be crosslinked, *e.g.,* with a cross-linker. Exemplary cross-linkers include glutaraldehyde (see, *e.g*., U.S. Pat. Nos. 4,852,640, 5,428,022, 5,660,692 and 5,008,116 1,4-butanediol diglycidyl ether, and genipin (see, *e.g.,* U.S. Patent Application Publication No. 2003/0049301). Further exemplary cross-linkers and methods of cross-linking collagen are described in U.S. Pat. Nos. 5,880,242 and 6,117,979 and in Zeeman et al., 2000, J Biomed Mater Res. 51(4):541-8, van Wachem et al., 2000, J Biomed Mater Res. 53(1):18-27, van Wachem et al., 1999, J Biomed Mater Res. 47(2):270-7, Zeeman et al., 1999, J Biomed Mater Res. 46(3):424-33, Zeeman et al., 1999, Biomaterials 20(10):921-31.

### 4.1.1 Formulations

The ECM compositions provided herein can be formulated in multiple ways, and the type of formulation can be selected based on, *e.g.,* the intended use of the ECM composition.

In a specific embodiment, the ECM compositions provided herein are formulated as a flowable matrix, *e*.*g*., in a form that can be administered using a syringe. Presented herein, therefore, is a syringe comprising an ECM composition as described herein. The flowable matrix ECM compositions provided herein can be formulated in water or phosphate buffered saline, *e.g.,* as a solution or suspension, *e.g.,* a mouthwash. When in solution (*i*.*e*., as a flowable matrix), an ECM composition provided herein can be present at any concentration useful to one of skill in the art. A flowable matrix ECM composition provided herein may comprise 200-300 mg/ml, 100-200 mg/ml, 150-250 mg/ml, 0.1-100 mg/ml, 1-100 mg/ml, 1-75 mg/ml, 1-50 mg/ml, 1-40 mg/ml, 10-40 mg/ml or 20-40 mg/ml of ECM. A flowable matrix ECM composition provided herein may comprise about 5 mg/ml, 10 mg/ml, 15 mg/ml, 20 mg/ml, 25 mg/ml, 30 mg/ml, 35 mg/ml, 40 mg/ml, 45 mg/ml, 50 mg/ml ECM, 75 mg/ml, 100 mg/ml, 125 mg/ml, 150 mg/ml, 175 mg/ml, 200 mg/ml, 225 mg/ml, 250 mg/ml, 275 mg/ml, or 300 mg/ml ECM. Disclosed herein is a flowable matrix ECM composition comprising about 200 mg/ml ECM. A flowable matrix ECM composition provided herein may be prepared by one of skill in the art using lyophilized, micronized ECM prepared using a method described herein, *e*.*g*., said lyophilized, micronized ECM is provided in the form of a kit, accompanied by an appropriate suspension solution, *e*.*g*., saline, and one of skill in the art can easily generate a flowable matrix ECM composition by suspending the lyophilized, micronized ECM in the suspension solution.

In another specific embodiment, the ECM compositions provided herein are formulated as a particulate, *e*.*g*., in powder form. When present as a particulate, the ECM compositions provided herein can be provided in any container suitable for storage of a particulate, *e*.*g*., a vial *(e.g.,* a glass vial). When provided as a particulate, an ECM composition provided herein can be provided in a container at any concentration useful to one of skill in the art. A particulate ECM composition provided herein may comprise 200-300 mg, 100-200 mg, 150-250 mg, 50-100 mg, 25-50 mg, 10-25 mg, 5-10 mg, or 1-5 mg of ECM. A flowable matrix ECM composition provided herein may comprise about 5 mg, 10 mg, 15 mg, 20 mg, 25 mg, 30 mg, 35 mg, 40 m, 45 mg, 50 m ECM, 75 mg, 100 mg, 125 mg, 150 mg, 175 mg, 200 mg, 225 mg, 250 mg, 275 mg, or 300 mg ECM. Descrbied herein is a particulate ECM composition comprising about 200 mg ECM. Described herein is a particulate ECM composition comprising about 100 mg ECM

In another specific embodiment, the ECM compositions provided herein are formulated as a sheet, *i.e*., a planar, solid layer of ECM. When present as a sheet, the ECM compositions provided herein can be shaped into a sheet of any thickness and dimensions suitable for their intended use. The ECM sheets provided herein may be provided in a standard size, *e.g.,* 5 x 5 cm or 8 x 8 cm in size, and can be manipulated *(e.g.,* cut) by one of skill in the art prior to their use, *e.g.,* cut such that they are of suitable size for their intended use, *e.g*., as a wound dressing. The ECM compositions provided herein may be formulated as a sheet having a thickness of about 0.1-0.15 mm, 0.15-0.2 mm, 0.1-0.2 mm, 0.2-0.25 mm, 0.25-0.3 mm, or 0.2-0.3 mm. The ECM compositions provided herein may be formulated as a sheet having a thickness of about 0.1 mm, 0.15 mm, 0.2 mm, 0.25 mm, or 0.3 mm. The ECM compositions provided herein may be formulated as a 2 x 2 cm sheet, a 3 x 3 cm sheet, a 4 x 4 cm sheet, a 5 x 5 cm sheet, a 6 x 6 cm sheet, a 7 x 7 cm sheet, a 8 x 8 cm sheet, or a 9 x 9 cm sheet, wherein said sheet has a thickness of about 0.1-0.15 mm, 0.15-0.2 mm, 0.1-0.2 mm, 0.2-0.25 mm, 0.25-0.3 mm, or 0.2-0.3 mm.

Described herein are laminates comprising at least one ECM sheet provided herein. Described herein is a laminate comprising two ECM sheets. Described herein is a laminate comprising at least one ECM sheet provided herein and at least one other planar decellularized tissue (*e*.*g*., decellularized/dehydrated amniotic membrane, either completely decellularized or decellularized so as to retain a fibroblastic cell layer), or with a planar artificial tissue substitute. Laminates can be generated by placing ECM sheets, or one or more ECM sheets and another planar decellularized tissue, in contact with each other in the presence of an adhesive, *e.g.,* a glue *(e.g.,* natural glue, *e.g.,* fibronectin, fibrin; or synthetic glue). Laminates can be generated by heat-drying together two or more ECM sheets, or one or more ECM sheets and one or more planar decellularized tissues.

### 4.1.1.1 Non-ECM Components

An ECM composition provided herein may be formulated to comprise one or more components that are not normally associated with ECM.

An ECM composition provided herein may be combined with a pharmaceutically or cosmetically acceptable carrier, such that the ECM composition is suitable for administration to a subject, *e.g.,* a human subject in need of such administration. Forms of administration include, but are not limited to, injections, solutions, creams, gels, implants, pumps, ointments, emulsions, suspensions, microspheres, particles, microparticles, nanoparticles, liposomes, pastes, patches, tablets, transdermal delivery devices, sprays, aerosols, and other means known to one of skill in the art. Such pharmaceutically or cosmetically acceptable carriers are commonly known to one of ordinary skill in the art. The terms "pharmaceutically or cosmetically acceptable carrier" or "pharmaceutically or cosmetically acceptable vehicle" are used herein to mean, without limitations, any liquid, solid or semi-solid, including, but not limited to, water or saline, a gel, cream, salve, solvent, diluent, fluid ointment base, ointment, paste, implant, liposome, micelle, giant micelle, and the like, which is suitable for use in contact with living animal or human tissue without causing adverse physiological or cosmetic responses, and which does not interact with the other components of the composition, *e*.*g*., the ECM, in a deleterious manner.

An ECM composition provided herein may be constituted such that is capable of releasing an active ingredient, *e*.*g*., an active ingredient in addition to the ECM composition. For example, an ECM composition provided herein may be impregnated, either during production or afterward, with a biomolecule. Exemplary biomolecules include, but are not limited to, antibiotics (such as clindamycin, minocycline, doxycycline, gentamycin), hormones, growth factors, anti-tumor agents, anti-fungal agents, anti-viral agents, pain medications, anti-histamines, anti-inflammatory agents, anti-infectives, elemental silver, antibiotics, bactericidal enzymes (such as lysozome), wound healing agents (such as cytokines including but not limited to PDGF, TGF; thymosin), hyaluronic acid, wound sealants (such as fibrin with or without thrombin), and cellular attractant and scaffolding reagents. In a specific example, an ECM composition provided herein is impregnated with at least one growth factor, for example, fibroblast growth factor or epithelial growth factor. An ECM composition provided herein can be impregnated with small organic molecules, such as specific inhibitors of particular biochemical processes *e*.*g*., membrane receptor inhibitors, kinase inhibitors, growth inhibitors, and anticancer drugs.

An ECM composition provided herein may be combined with a hydrogel. Any hydrogel known to one skilled in the art may be used, *e.g.,* any of the hydrogel compositions disclosed in Graham, 1998, Med. Device Technol. 9(1): 18-22; Peppas et al., 2000, Eur. J. Pharm. Biopharm. 50(1): 27-46; Nguyen et al., 2002, Biomaterials, 23(22): 4307-14; Henincl et al., 2002, Adv. Drug Deliv. Rev 54(1): 13-36; Skelhorne et al., 2002, Med. Device. Technol. 13(9): 19-23; or Schmedlen et al., 2002, Biomaterials 23: 4325-32. The hydrogel may be applied onto the ECM composition, *i*.*e*., discharged on the surface of the ECM composition. The hydrogel for example, may be sprayed onto the ECM composition, saturated on the surface of the ECM composition, soaked with the ECM composition, bathed with the ECM composition, or coated onto the surface of the ECM composition. The hydrogels useful in the methods and compositions provided herein can be made from any water-interactive, or water soluble polymer known in the art, including but not limited to, polyvinylalcohol (PVA), polyhydroxyehthyl methacrylate, polyethylene glycol, polyvinyl pyrrolidone, hyaluronic acid, dextran, and derivatives and analogs thereof.

### 4.1.2 Cells

The ECM compositions provided herein can comprise, be seeded with, or be cultured with one or more types of cells. For example, cells can be cultured with and grown upon an ECM composition described herein or dispersed on or within an ECM composition described herein *(e.g.,* added to an ECM flowable matrix composition). One of skill in the art will appreciate that any cell type known in the art can be seeded with and/or cultured with the ECM compositions provided herein, including both stem cells and non-stem cells.

The ECM compositions provided herein may be seeded with and/or comprise stem cells. The stem cells can be any stem cells suitable for a given purpose, and can be totipotent or pluripotent stem cells, or can be progenitor cells. An ECM composition provided herein may be seeded with and/or may comprise placental stem cells, such as those described in U.S. Patent Nos. 7,045,148; 7,468,276; 8,057,788 and 8,202,703. An ECM composition provided herein may be seeded with and/or may comprise embryonic stem cells, embryonic germ cells, mesenchymal stem cells, bone marrow-derived stem cells, hematopoietic progenitor cells (*e*.*g*., hematopoietic stem cells from peripheral blood, fetal blood, placental blood, umbilical cord blood, placental perfusate, etc.), somatic stem cells, neural stem cells, hepatic stem cells, pancreatic stem cells, endothelial stem cells, cardiac stem cells, muscle stem cells, adipose stem cells, and the like. The stem cells may be human stem cells.

The ECM compositions provided herein may be seeded with and/or may comprise one or more types of non-stem cells. As used herein, "non-stem cell" refers to a terminally-differentiated cell. For example, the ECM compositions provided herein may comprise a plurality of fibroblasts. Non-stem cells that can be combined with the ECM compositions provided herein include, without limitation, fibroblasts or fibroblast-like cells, dermal cells, endothelial cells, epithelial cells, muscle cells, cardiac cells and pancreatic cells. An ECM composition provided herein may be seeded with and/or may comprise at least two types of non-stem cells.

The ECM compositions provided herein may be cultured with cells for a time sufficient for a plurality of said cells to attach to the ECM composition. In accordance with such examples, the ECM composition can be shaped into a useful configuration, *e*.*g*., shaped as a sheet, plug, tube, or other configuration, prior to contacting the ECM composition with the cells.

The ECM compositions can be cultured with at least 1 X 10⁶, 3 X 10⁶, 1 X 10⁷, 3 X 10⁷, 1 X 10⁸, 3 X 10⁸, 1 X 10⁹, 3 X 10⁹, 1 X 10¹⁰, 3 X 10¹⁰, 1 X 10¹¹, 3 X 10¹¹, or 1 X 10¹²; or may be no more than 1 X 10⁶, 3X 10⁶, 1 X 10⁷, 3 X 10⁷, 1 X 10⁸, 3 X 10⁸, 1 X 10⁹,3 X 10⁹, 1 X 10¹⁰, 3 X 10¹⁰, 1 X 10¹¹, 3 X 10¹¹, or 1 X 10¹² cells.

### 4.1.1. Characterization

Biochemical based assays known in the art may be used to confirm the biochemical compositions of the ECM compositions produced using the methods described herein. Protein content can be determined using, *e*.*g*., absorbance assays, such as those described in Layne, E, Spectrophotometric and Turbidimetric Methods for Measuring Proteins, Methods in Enzymology 3: 447-455, (1957); Stoscheck, C M, Quantitation of Protein, Methods in Enzymology 182: 50-69, (1990)), Scopes, R K, Analytical Biochemistry 59: 277, (1974); and Stoscheck, C M. Quantitation of Protein, Methods in Enzymology 182: 50-69, (1990)). Alternatively, colorimetric based assays can be used to measure content of particular proteins, included including the modified Lowry assay, biuret assay, Bradford assay, and Bicinchoninic Acid (Smith) assay (see, *e*.*g*., Stoscheck, C M, Quantitation of Protein, Methods in Enzymology 182: 50-69 (1990)).

Total collagen content of the ECM compositions provided herein can be determined using, *e.g.,* hydroxyproline or a quantitative dye-based assay kit, *e.g.,* the SIRCOL^{™} kit manufactured by Biocolor Ltd, UK. Collagen types in the ECM compositions provided herein can be determined using standard methods known in the art, *e.g.,* ELISA assay.

Total elastin content of the ECM compositions provided herein can be determined using, *e.g.,* a quantitative dye-based assay, *e.g.,* the dye-based assay kit (FASTIN) manufactured by Biocolor Ltd, UK.

Total glycosaminoglycan (GAG) content of the ECM compositions provided herein can be determined using, *e.g.,* the quantitative dye-based assay kit (BLYSCAN) manufactured by Biocolor Ltd, UK. GAG content also can be measured by ELISA, using methods known in the art.

Total laminin and fibronectin content of the ECM compositions provided herein can be determined using, for example, an ELISA assay, *e.g.,* a sandwich ELISA assay, *e.g.,* the ELISA assays specific for laminin or fibronectin provided as a kit from Takara Bio Inc., Shiga, Japan.

The ECM compositions described herein are non-immunogenic and biocompatible with tissues of subjects, *e*.*g*., human subjects. "Biocompatibility," as used herein refers to the property of being biologically compatible by not producing a toxic, injurious, or immunological response or rejection in living tissue. Biocompatibility assays can be performed to confirm biocompatibility of the ECM compositions provided herein. Such assays are known to one of skill in the art and include, but are not limited to, cytotoxicity assays (*e*.*g*., the ISO MEM Elution test), rabbit eye irritation tests, hemolysis assays, and pyrogencity assays.

The ECM compositions provided herein can be formulated to be sterile, and thus free of microbiological contaminants. Presence of microorganisms can be determined using art-known methods, *e*.*g*., direct inoculation of an ECM composition in/on an appropriate bacterial growth medium, *e*.*g*., soy casein media or thioglycollate media.

The ECM compositions provided herein can be formulated to comprise little, no, or undetectable levels of endotoxin. Presence of endotoxin in an ECM composition provided herein can be determined using, *e.g.,* the Limulus Amebocyte Lysate (LAL) test (bacterial endotoxin test), an in vitro assay well-known in the art. The ECM compositions provided herein may comprise less than 20 endotoxin units (EU) per formulation (*e*.*g*., a sheet of ECM provided herein comprises less than 20 EU).

### 4.2. Methods of Making Extracellular Matrix Compositions

In one aspect, provided herein are methods of generating placental extracellular matrix (ECM) compositions. The methods of making ECM compositions from placenta (*e*.*g*., human placenta) that are provided herein may comprise the following steps, in order: (i) removing the amnion, chorion, and umbilical cord from a placenta (*e*.*g*., from a placenta obtained from a mother immediately after birth, or from a stored placenta); (ii) subjecting the placental tissue to a solution that causes osmotic disruption of cells associated with the placental tissue; (iii) contacting the placenta with a solution comprising a detergent; and (iv) contacting the placenta with a solution comprising a base. The methods of making ECM compositions from placenta may use the chorion of the placenta (*e*.*g*., human placenta), wherein said methods comprise the following steps, in order: (i) obtaining the chorion from a placenta *(e.g.,* from a placenta obtained from a mother immediately after birth, or from a stored placenta); (ii) subjecting the chorion to a solution that causes osmotic disruption of cells associated with the chorion; (iii) contacting the chorion with a solution comprising a detergent; and (iv) contacting the chorion with a solution comprising a base. The methods of making ECM compositions from placenta may use the chorion of the placenta (*e*.*g*., human placenta), wherein said methods comprise the following steps, in order: (i) obtaining the chorion from a placenta *(e.g.,* from a placenta obtained from a mother immediately after birth, or from a stored placenta); (ii) scraping and cleaning the chorion; (iii) subjecting the chorion to a solution that causes osmotic disruption of cells associated with the chorion; (iv) contacting the chorion with a solution comprising a detergent; and (v) grinding and freeze drying. The methods of making ECM compositions from placenta may use the chorion of the placenta (*e*.*g*., human placenta), wherein said methods comprise the following steps, in order: (i) obtaining the chorion from a placenta (*e*.*g*., from a placenta obtained from a mother immediately after birth, or from a stored placenta); (ii) scraping and cleaning the chorion; (iii) subjecting the chorion to a solution that causes osmotic disruption of cells associated with the chorion; (iv) contacting the chorion with a solution comprising with a first, then a second detergent solution, said solutions comprising a detergent and a chelating agent, *e.g.,* EDTA; and (v) freeze drying. Importantly, the methods of making the ECM compositions described herein use components, *e.g.,* base, detergent, chelating agent, in amounts that result in the generation of ECM compositions having the particular characteristics of those described herein.

The placentas used in the methods of ECM generation provided herein are generally obtained following a full-term birth, and can be freshly isolated or previously isolated and stored frozen. Generally, when a frozen placenta is used to prepare an ECM composition in accordance with the methods described herein, the placenta is thawed, *e*.*g*., at room temperature before use. For example, the placenta can be thawed at ~22-23°C for ~24 hours, or until the placenta is ready for use.

Prior to preparing an ECM composition from placenta in accordance with the methods provided herein, the placenta may be exsanguinated, *i*.*e*., drained of the cord blood remaining after birth. The placenta may be 70% exsanguinated, 80% exsanguinated, 90% exsanguinated, 95% exsanguinated or 99% exsanguinated before use in a method provided herein.

The step of contacting the placental tissue (*e*.*g*., placental tissue from which the amnion, chorion, and umbilical cord has been removed; or chorion from placenta) with a solution that causes osmotic disruption of cells associated with the placental tissue being processed in the methods provided herein results in removal of blood and blood components as well as cells and cellular debris that are normally associated with placental tissue. Accordingly, one of skill in the art will understand that any solution capable of causing osmotic disruption of cells can be used in the methods described herein. For example, NaCl, potassium chloride (KCl), ammonium sulfate, a monosaccharide, a disaccharide *(e.g.,* 20% sucrose), a hydrophilic polymer (*e*.*g*., polyethylene glycol), glycerol can be used to disrupt cells due to their osmotic potential.

NaCl is used to cause osmotic disruption of cells associated with the placental tissue (*e*.*g*., placental tissue from which the amnion, chorion, and umbilical cord has been removed; or chorion from placenta) used in the methods described herein. A solution comprising about 0.5M, or 1.0M, NaCl is used to cause osmotic disruption of cells associated with the placental tissue used in the methods described herein.

The step of contacting the placental tissue (*e*.*g*., placental tissue from which the amnion, chorion, and umbilical cord has been removed; or chorion from placenta) with a solution that causes osmotic disruption can be carried out at any temperature according to the judgment of one of skill in the art. The step may be carried out at about 0°C to 30°C, about 5°C to 25°C, about 5°C to 20°C, or about 5°C to 15°C. The step may be carried out at about 0°C, about 5°C, about 10°C, about 15°C, about 20°C, about 25°C, or about 30°C. The step may be carried out at room temperature. The step may be carried out at 37°C ± 5°C.

The step of contacting the placental tissue (*e*.*g*., placental tissue from which the amnion, chorion, and umbilical cord has been removed; or chorion from placenta) with a solution that causes osmotic disruption can be carried out for a suitable time according to the judgment of those of skill in the art. The step can be carried out for about 1-24 hours, about 2-20 hours, about 5-15 hours, about 8-12 hours, or about 2-5 hours. The step may be carried out for about 18-26 hours at room temperature. The step may be carried out for about 18-26 hours at 37°C ± 5°C.

The step of contacting placental tissue (*e*.*g*., placental tissue from which the amnion, chorion, and umbilical cord has been removed; or chorion from placenta) with a detergent results in removal of cells and cellular debris *(e.g.,* cell membranes) as well as the removal of nucleic acids from the ECM compositions provided herein. Accordingly, the detergent used in the methods described herein can be any detergent known to one of skill in the art to be capable of disrupting cellular or subcellular membranes. The detergent is ionic. The detergent is sodium deoxycholate or deoxycholic acid. In certain embodiments (which are not claimed), the detergent is zwitterionic. In certain embodiments (which are not claimed), the detergent is nonionic. For instance, in certain embodiments (which are not claimed), the detergent can be a TWEEN^{®} detergent, such as TWEEN^{®}-20, or a Triton X detergent, such as Triton X 100. The collagen composition can be contacted with the detergent under conditions judged by one of skill in the art to be suitable for removing unwanted components from the composition.

The detergent used in the methods provided herein is sodium deoxycholate or deoxycholic acid. Said sodium deoxycholate or deoxycholic acid can be used in a method described herein at a suitable concentration for removing cells, cellular debris, and nucleic acid. In particular embodiments of the methods described herein, the sodium deoxycholate or deoxycholic acid can be used, at a final concentration of 0.05-0.1% or 2%. In a specific embodiment, said sodium deoxycholate or deoxycholic acid is used in a method described herein at a final concentration of about 2%.

In particular other embodiments of the methods described herein, for example, methods that utilize the detergent, *e.g.,* the sodium deoxycholate or deoxycholic acid, together with a chelating agent, for example ethylenediaminetetraacetic acid (EDTA), in a first detergent solution, such sodium deoxycholate or deoxycholic acid is used; at a final concentration of about 0.05 to about 0.1%. The sodium deoxycholate or deoxycholic acid can be used at a final concentration of about 0.067%. With respect to the chelating agent present in such a first detergent solution, the chelating agent is EDTA, said EDTA is used, at a final concentration of about 3mM to about 5mM. In a specific example of the first detergent solution, the solution can comprise sodium deoxycholate or deoxycholic acid at a final concentration of about 0.067% and EDTA at a final concentration of about 4mM.

In examples of the methods described herein, for example, methods that utilize the detergent, *e.g*., the sodium deoxycholate or deoxycholic acid, together with a chelating agent, for example EDTA, in a second detergent solution, such sodium deoxycholate or deoxycholic acid can be used, *e.g*., a final concentration of about 0.05 to about 0.1%, about 0.05% to about 0.2%, about 0.2 to about 0.3%, about 0.3% to about 0.4%, or about 0.4 to about 0.5%; or at a final concentration of about 0.05%, about 0.06%, about 0.07%, about 0.08%, about 0.09%, about 0.1%, about 0.2%, about 0.3%, about 0.4%, or about 0.5%. The sodium deoxycholate or deoxycholic acid can be used at a final concentration of about 0.39%. With respect to the chelating agent present in such a second detergent solution, when the chelating agent is EDTA, such EDTA can be used, *e.g.,* at a final concentration of about 1 to about 5 mM, about 2 to about 5 mM, about 2 to about 4mM, about 3 to about 4mM, about 3mM to about 5mM. about 5 to about 10 mM, about 6 to about 10 mM, about 7 to about 9mM, or about 8 to about 10mM; or about 1mM, about 2mM, about 3mM, about 4mM, about 5mM, about 6mM, about 7mM, about 8mM, about 9mM or about 10mM. In a specific embodiment of the first detergent solution (which is not claimed), the solution can comprise sodium deoxycholate or deoxycholic acid at a final concentration of about 0.39% and EDTA at a final concentration of about 8mM.

The step of contacting placental tissue (*e*.*g*., placental tissue from which the amnion, chorion, and umbilical cord has been removed; or chorion from placenta) with a detergent can be carried out at any temperature according to the judgment of one of skill in the art. The detergent treatment may be carried out at about 0°C to 30°C., about 5°C to 25°C, about 5°C to 20°C, or about 5°C to 15°C. The detergent treatment step may be carried out at about 0°C, about 5°C, about 10°C, about 15°C, about 20°C, about 25°C, or about 30°C. The detergent treatment step may be carried out at room temperature. The detergent treatment step may be carried out at 37°C ± 5°C.

The detergent treatment (with or without a chelating agent) can be carried out for a suitable time according to the judgment of one of skill in the art. The detergent treatment can be carried out for about 1-24 hours, overnight, about 24-48 hours, or about 48-72 hours. The detergent treatment step may be carried out for about 72 hours. The detergent treatment step may be carried out for about 72 hours at room temperature. The detergent treatment step may be carried out for about 72 hours at 37°C ± 5°C.

The step of contacting placental tissue (*e*.*g*., placental tissue from which the amnion, chorion, and umbilical cord has been removed; or chorion from placenta) with a base results in removal of certain ECM components from the ECM compositions provided herein, *e*.*g*., removal of fibronectin and laminin, by denaturing such ECM components. Exemplary bases for the basic treatment include biocompatible bases, volatile bases or bases known to those of skill in the art to be easily and safely removed from the ECM. The base can be any organic or inorganic bases known to those of skill in the art at a concentration of, for example, 0.2M to 1.0M. The base may be ammonium hydroxide, potassium hydroxide or sodium hydroxide, *e*.*g*., an ammonium hydroxide solution, potassium hydroxide solution or sodium hydroxide solution.

In a specific embodiment, the base used in the base treatment step of the methods provided herein is sodium hydroxide (NaOH). NaOH can be used in the methods described herein at any concentration suitable for removal of, *e.g.,* laminin and fibronectin from the ECM compositions provided herein. For example, NaOH can be used in a method described herein at a concentration of about 0.1M NaOH, 0.25M NaOH, 0.5M NaOH, 1M NaOH, 1.5M NaOH, or 2M NaOH. In a specific embodiment, NaOH is used in a method described herein at a concentration of about 1M NaOH.

The base treatment step can be carried out at any temperature according to the judgment of one of skill in the art. The basic treatment may be carried out at about 0°C to 30°C, about 5°C to 25°C, about 5°C to 20°C, or about 5°C to 15°C. The basic treatment may be carried out at about 0°C, about 5°C, about 10°C, about 15°C, about 20°C, about 25°C, or about 30°C. The base treatment step may be carried out at room temperature. The base treatment step may be carried out at 37°C ± 5°C.

The base treatment step can be carried out for a suitable time according to the judgment of one of skill in the art. The base treatment step can be carried out for about 15 minutes to 30 minutes, 30 minutes to 1 hour, 1 to 2 hours, 2 to 4 hours, 4-8 hours, 8-12 hours, or about 12-24 hours. The base treatment step may be carried out for about 30 minutes. The base treatment step may be carried out for about 30 minutes at room temperature. The base treatment step may be carried out for about 30 minutes at 37°C ± 5°C.

Any or all of the steps of the methods described herein may be carried out under sterile conditions. The ECM compositions prepared according to the methods described herein may be further sterilized according to techniques apparent to one of skill in the art and described below.

In a specific embodiment, provided herein is a method of making an ECM composition comprising 35-55% collagen by dry weight and 10-30% elastin by dry weight, said method comprising (i) removing the amnion, chorion, and umbilical cord from a placenta (e.g., from a placenta obtained from a mother immediately after birth, or from a stored placenta); (ii) placing the remaining placental tissue in a solution comprising 1 M NaCl and homogenizing the placental tissue; (iii) contacting the placental tissue with a solution comprising 2% sodium deoxycholate; (iv) washing the placental tissue, with water; (v) contacting the placental tissue with a solution comprising 1 M NaOH; (vi) adding an acid solution, *e*.*g*., hydrochloric acid (HCl), to the solution comprising placental tissue to bring it to a neutral pH *(e.g.,* pH 6.0-8.0); and (vii) separating the placental tissue from the liquid portion of the solution (*e*.*g*., by centrifugation) and collecting the placental tissue, thereby making an ECM composition. The ECM composition generated according to the method generally is in the form of a paste (ECM paste), which can be frozen and stored after collection for later use, or which can be used directly after collection to manufacture an ECM formulation described herein, *e.g*., in the formulation of an ECM sheet, an ECM particulate formulation, or an ECM flowable matrix.

Described herein is a method of making an ECM composition, said method comprising (i) removing the amnion, chorion, and umbilical cord from a placenta (*e*.*g*., from a placenta obtained from a mother immediately after birth, or from a stored placenta); (ii) cutting the placenta into strips, *e.g.,* 2 x 2 centimeter strips; (iii) placing the placental tissue in a 1.5 liter 1 M NaCl solution and homogenizing the placental tissue (*e*.*g*., using an Omni Mixer Homogenizer (Omni International, Kennesaw, GA)), (iv) placing the homogenized placental tissue in a processing receptacle *(e.g.,* a bag) and adding 1 M NaCL to a volume of 9.2 liters; (v) washing the homogenized placental tissue three times with 1 M NaCl, wherein said washing comprises (a) agitating the processing receptacle on a shaker for 10 minutes, (b) allowing placental tissue to settle in the processing receptacle for 10 minutes, and (c) removing 6.2 liters of the supernatant by gravity drainage; (vi) allowing the washed placental tissue to mix in the remaining 1 M NaCl solution on a shaker for ∼18-26 hours at room temperature (vii) washing the placental tissue four times with water, as described above; (viii) allowing the washed placental tissue to mix in water on a shaker for ~18-26 hours at room temperature; (ix) after the overnight mixing in water, washing a final time with water, as described above, then adding 6.2 L of 3% sodium deoxycholate to the mixture, for a concentration of 2% sodium deoxycholate; (x) allowing the placental tissue to mix in the 2% sodium deoxycholate solution on a shaker for ∼72 hours at room temperature; (xi) washing the placental tissue in water five times, in the manner described above; (xii) after the final addition of water, adjusting the pH of the solution to about 10-12 by addition of 1M NaOH, resulting in a basic solution; (xiii) allowing the placental tissue to mix in the basic solution on a shaker for ~30 minutes at room temperature; (xiv) adjusting the pH of the solution to about 7.0-7.5 using 0.1 N HCl; and (xv) removing the supernatant from the processing receptacle (*e*.*g*., by centrifugation) and collecting the placental tissue, thereby making an ECM composition.

Described herein is a method of making an ECM composition, said method comprising (i) obtaining the chorion from a placenta (*e*.*g*., from a placenta obtained from a mother immediately after birth, or from a stored placenta); (ii) placing the placental chorion in a solution comprising 1 M NaCl and homogenizing the placental chorion; (iii) contacting the placental chorion tissue with a solution comprising 2% sodium deoxycholate; (iv) washing the placental chorion tissue, with water; (v) contacting the placental chorion tissue with a solution comprising 1 M NaOH; (vi) adding an acid solution, *e*.*g*., hydrochloric acid (HCl), to the solution comprising placental chorion tissue to bring it to or close to a neutral pH (*e*.*g*., pH 6.0-8.0); and (vii) separating the placental chorion tissue from the liquid portion of the solution (*e*.*g*., by centrifugation) and collecting the placental chorion tissue, thereby making an ECM composition. The ECM composition generated according to the method generally is in the form of a paste (ECM paste), which can be frozen and stored after collection for later use, or which can be used directly after collection to manufacture an ECM formulation described herein, *e*.*g*., in the formulation of an ECM sheet, an ECM particulate formulation, or an ECM flowable matrix. The various steps of the method (*e*.*g*., osmotic disruption, detergent treatment, washing, base treatment) may be carried out at room temperature. The various steps of the method (e.g., osmotic disruption, detergent treatment, washing, base treatment) may be carried out at 37°C ± 5°C. The washing step may be carried out using a volume of ½ to 2 liters of fluid for 1-3 times.

Provided herein is a method of making an ECM composition, said method comprising (i) obtaining the chorion from a placenta (*e*.*g*., from a placenta obtained from a mother immediately after birth, or from a stored placenta); (ii) cutting the chorion into strips, *e*.*g*., 2 x 2 centimeter strips; (iii) placing the placental chorion tissue in a 1.5 liter 1 M NaCl solution and homogenizing the placental chorion tissue (*e*.*g*., using an Omni Mixer Homogenizer (Omni International, Kennesaw, GA)), (iv) placing the homogenized placental chorion tissue in a processing receptacle *(e.g.,* a bag) and adding 1 M NaCL to a volume of 9.2 liters; (v) washing the homogenized placental chorion tissue three times with 1 M NaCl, wherein said washing comprises (a) agitating the processing receptacle on a shaker for 10 minutes, (b) allowing placental chorion tissue to settle in the processing receptacle for 10 minutes, and (c) removing 6.2 liters of the supernatant by gravity drainage; (vi) allowing the washed placental chorion tissue to mix in the remaining 1 M NaCl solution on a shaker for ~18-26 hours at room temperature (vii) washing the placental chorion tissue four times with water, as described above; (viii) allowing the washed placental chorion tissue to mix in water on a shaker for ∼18-26 hours at room temperature; (ix) after the overnight mixing in water, washing a final time with water, as described above, then adding 6.2 L of 3% sodium deoxycholate to the mixture, for a concentration of 2% sodium deoxycholate; (x) allowing the placental chorion tissue to mix in the 2% sodium deoxycholate solution on a shaker for ∼72 hours at room temperature; (xi) washing the placental chorion tissue in water five times, in the manner described above; (xii) after the final addition of water, adjusting the pH of the solution to about 10-12 by addition of 1M NaOH, resulting in a basic solution; (xiii) allowing the placental chorion tissue to mix in the basic solution on a shaker for ∼30 minutes at room temperature; (xiv) adjusting the pH of the solution to about 7.0-7.5 using 0.1 N HCl; and (xv) removing the supernatant from the processing receptacle (*e*.*g*., by centrifugation) and collecting the placental chorion tissue, thereby making an ECM composition. The washing step may be carried out using a volume of ½ to 2 liters of fluid.

In another specific embodiment, provided herein is a method of making an ECM composition comprising 40-70% collagen by dry weight and 15-25% elastin by dry weight, said method comprising (i) obtaining the chorion, for example, chorionic plate, from a placenta (*e*.*g*., from a placenta obtained from a mother immediately after birth, or from a stored placenta); (ii) scraping and cleaning the chorion; (iii) placing the chorion tissue in a solution comprising 0.5 M NaCl; (iv) contacting the chorion tissue with a solution comprising a 2% deoxycholic acid or sodium deoxycholate and rinsing by water; and (v) grinding and freeze drying. The ECM composition, generally a paste (ECM paste) can be formulated, for example, milled and formulated, into a variety of shapes and forms, *e.g.,* an ECM sheet, an ECM particulate formulation, or an ECM flowable matrix. The various steps of the method (*e*.*g*., osmotic disruption, detergent treatment, rinsing) may be carried out at room temperature. The various steps of the method (*e*.*g*., osmotic disruption, detergent treatment, rinsing) may be carried out at 37°C ± 5°C. The rinsing step may be carried out using a volume of ½ to 2 liters of fluid for 1-3 times.

Described herein is a method of making an ECM composition, said method comprising, in order: (i) obtaining a human placenta from a mother immediately after a full-term birth, or obtaining a previously isolated frozen human placenta that has been allowed to thaw, for example, allowed to thaw at room temperature for approximately 24 hours; (ii) washing the placenta is washed in 0.5M NaCl; (iii) removing the amnion, umbilical cord and decidua parietalis from the placenta, and retaining the chorionic plate of the placenta; (iv) scraping and cleaning the chorion; (v) rinsing the chorion in 0.5 M NaCl and water; (vi) rinsing the chorion overnight in 2% deoxycholic acid, followed by multiple water rinses, *e.g.,* 3, 4, 5, 6, 7 or more water rinses; and (vii) grinding and freeze drying the treated chorion. The ECM composition, generally a paste (ECM paste) can be formulated, for example, milled and formulated, into a variety of shapes and forms, *e.g.,* an ECM sheet, an ECM particulate formulation, or an ECM flowable matrix. The various steps of the method (*e*.*g*., NaCl treatment, deoxycholic acid treatment, rinsing) may be carried out at room temperature. The various steps of the method (*e*.*g*., NaCl treatment, deoxycholic acid treatment, rinsing) may be carried out at 37°C ± 5°C. The rinsing step may be carried out using a volume of ½ to 2 liters of fluid for 1-3 times.

Described herein is a method of making an ECM composition comprising 40-70% collagen by dry weight and 15-25% elastin by dry weight, said method comprising (i) obtaining the chorion, for example, chorionic plate, from a placenta (*e*.*g*., from a placenta obtained from a mother immediately after birth, or from a stored placenta); (ii) scraping and cleaning the chorion; (iii) placing the chorion tissue in a solution 1.0 M NaCl; (iv) contacting the chorion tissue with a first detergent solution comprising 0.05%-0.1% deoxycholic acid or sodium deoxycholate and 3mM-5mM EDTA); (v) contacting the chorion tissue with a second detergent solution comprising a detergent 0.05-0.1% deoxycholic acid or sodium deoxycholate) and EDTA (*e*.*g*.,., 1-5mM EDTA or 5-10 mM EDTA), and by water; and (vi) freeze drying to yield a decellularized, freeze dried whole chorion which can be formulated, for example, milled into and resuspended in solution (e.g., water or phosphate-buffered saline) to form a decellularized ECM paste, and formulated, into a variety of shapes and forms, e.g., an ECM sheet, an ECM particulate formulation, or an ECM flowable matrix. The various steps of the method (*e*.*g*., osmotic disruption, detergent/EDTA treatment, rinsing) may be carried out at room temperature. The various steps of the method (*e*.*g*., osmotic disruption, detergent/EDTA treatment, rinsing) may be carried out at 37°C ± 5°C. The rinsing step may be carried out using a volume of ½ to 2 liters of fluid for 1-3 times.

Described herein is a method of making an ECM composition, said method comprising, in order: (i) obtaining a human placenta from a mother immediately after a full-term birth, or obtaining a previously isolated frozen human placenta that has been allowed to thaw, for example, allowed to thaw at room temperature for approximately 24 hours; (ii) removing the amnion, umbilical cord and decidua parietalis from the placenta, and retaining the chorionic plate of the placenta; (iii) scraping and cleaning the chorion; (iv) rinsing the chorion in 1.0 M NaCl and water; (v) rinsing the chorion overnight in a first detergent solution comprising 0.067% deoxycholic acid and 4mM EDTA, followed by multiple water rinses, *e.g.,* 3, 4, 5, 6, 7 or more water rinses; (vi) rinsing the chorion overnight in a second detergent solution comprising 0.39% deoxycholic acid and 8mM EDTA, followed by multiple water rinses, *e.g.,* 3, 4, 5, 6, 7 or more water rinses; and (vii) freeze drying the treated chorion. The resulting composition is a decellularized ECM paste suitable for further formulation, *e*.*g*., milling and formulation. The various steps of the method *(e.g.,* NaCl treatment, deoxycholic acid/EDTA treatment, rinsing) may be carried out at room temperature. The various steps of the method *(e.g.,* NaCl treatment, deoxycholic acid/EDTA treatment, rinsing) may be carried out at 37°C ± 5°C. The rinsing step may be carried out using a volume of ½ to 2 liters of fluid for 1-3 times.

Described herein, but not explicitly claimed, is a method of generating an ECM sheet, said method comprising (i) preparing an ECM paste according to the methods described herein; (ii) suspending the ECM paste in, *e*.*g*., water, and adding the suspended ECM solution to a suitable substrate for formation of a sheet, *e*.*g*., adding the ECM to a mold; (iii) freezing the ECM (iv) lyophilizing the frozen ECM; (v) removing the lyophilized ECM from the substrate and soaking it in water; and (vi) drying the ECM, *e.g.,* using a vacuum dryer. After rehydration, and prior to drying, the ECM sheets provided herein can be formed into any useful shape, *e.g.,* a block, a tube, or another shape.

Described herein, but not explicitly claimed, is a method of generating an ECM particulate, said method comprising (i) preparing an ECM paste according to the methods described herein; (ii) suspending the ECM paste in, *e.g*., water; (iii) freezing the ECM (iv) lyophilizing the frozen ECM; and (v) milling the lyophilized ECM.

Described herein, but not explicitly claimed, is a method of generating an ECM flowable matrix, said method comprising (i) preparing an ECM paste according to the methods described herein; (ii) suspending the ECM paste in, *e*.*g*., water; (iii) freezing the ECM (iv) lyophilizing the frozen ECM; and (v) micronizing the lyophilized ECM. Upon resuspension of the micronized ECM in, *e.g.,* saline, an ECM flowable matrix is generated.

Lyophilization of the ECM compositions produced according to the methods described herein can be accomplished by any means known in the art, and generally proceeds until the ECM composition is substantially dry, *e.g.,* less than about 30%, 25%, 20%, 25%, 20%, 5%, 4%, 3%, 2% or 1% water by weight.

### 4.2.1. Optional Further Treatment

The methods provided herein may incorporate an additional step, *e.g.,* a step that results in treatment of the ECM composition being prepared with another agent other than a solution capable of causing osmotic disruption of cells, a detergent, or a base.

The methods provided herein may comprise treatment of an ECM composition being prepared according to the methods described herein with BENZONASE^{®}. BENZONASE^{®} is a genetically engineered endonuclease derived from *Serratia marcescens* that attacks and degrades all forms of DNA and RNA. Accordingly, BENZONASE^{®} can be used in accordance with the methods described herein to ensure that the resulting ECM composition are free of (or substantially free of) nucleic acid. After treatment with BENZONASE^{®}, the ECM composition treated can be brought to a high pH, then a low pH, conditions suitable for inactivation of BENZONASE^{®}.

The methods provided herein may comprise treatment of an ECM composition being prepared according to the methods described herein with ethylenediaminetetraacetic acid (EDTA). EDTA is a metal chelator well-known to one of skill in the art, and can be used in accordance with the methods provided herein to remove divalent metal ions from the ECM compositions provided herein. EDTA can be washed out of the ECM compositions using methods known to one of skill in the art.

The collagen in the ECM compositions provided herein can be cross-linked. The cross-linking can be with any cross-linker known to one of skill in the art, for instance, the cross-linkers described above. The cross-linker may be glutaraldehyde, and the cross-linking can be carried out according to methods of glutaraldehyde cross-linking of collagen known to one of skill in the art. The cross-linker may be 1,4-butanediol diglycidyl ether or genipin.

### 4.2.2. Storage

The ECM compositions provided herein may be stored at room temperature (e.g., ~22-25°C). The ECM compositions provided herein may be stored cold, *e*.*g*., refrigerated at a temperature of about 0°C, about 4°C, or about 8°C. In some embodiments, the ECM is not refrigerated. The ECM compositions provided herein may be stored frozen, *i.e*., at a temperature below 0°C, *e.g.,* at -10°C, -15°C, -20°C, -25°C, -30°C, -35°C, -40°C, -45°C, -50°C, -55°C, -60°C, -65°C, -70°C, -75°C, -80°C, or -85°C, or colder. Said freezing and storage of the ECM compositions provided herein may take place at a temperature between 0°C to -10°C, -10°C to -20°C, -20°C to -30°C, -30°C to -40°C, -40°C to -50°C, -50°C to -60°C, -60°C to -70°C, or -70°C, to -80°C.

The ECM compositions provided herein may be stored under sterile and non-oxidizing conditions. The ECM compositions provided herein may be stored at any of the above-specified temperatures for 12 months or more.

### 4.2.3. Sterilization

The ECM compositions provided herein may be sterilized according to techniques known to those of skill in the art for sterilizing such compositions. The the ECM compositions provided herein may be sterilized by radiation, *e*.*g*., gamma irradiation.

Sterilization of the ECM compositions provided herein may be carried out by electron beam irradiation using methods known to one skilled in the art, see, *e*.*g*., Gorham, D. Byrom (ed.), 1991, Biomaterials, Stockton Press, New York, 55-122. Any dose of radiation sufficient to kill at least 99.9% of bacteria or other potentially contaminating organisms is within the scope of the methods provided herein. A dose of at least 18-25 kGy may be used to achieve terminal sterilization of an ECM composition provided herein.

The ECM compositions provided herein may be filtered through a filter that allows passage of endotoxins and retains the ECM composition. Any filter of a size, for example 30 kDa, known to one of skill in the art for filtration of endotoxins can be used. The filter may be between 5 kDa and 100 kDa, *e.g.,* the filter is about 5 kDa, about 10 kDa, about 15 kDa, about 20 kDa, about 30 kDa, about 40 kDa, about 50 kDa, about 60 kDa, about 70 kDa, about 80 kDa, about 90 kDa or about 100 kDa. The filter can be of any material known to those of skill in the art to be compatible with the ECM compositions provided herein, such as cellulose or polyethersulfone. The filtration can be repeated as many times as desired by one of skill in the art. Endotoxin can be detected according to standard techniques to monitor clearance.

The ECM compositions provided herein may be filtered to generate ECM compositions free of, or reduced in, viral particles. Any filter known to one of skill in the art to be useful for clearing viruses can be used. For instance, a 1000 kDa filter can be used for clearance, or reduction, of parvovirus, hepatitis A virus and HIV. A 750 kDa filter can be used for clearance, or reduction, of parvovirus and hepatitis A virus. A 500 kDa filter can be used for clearance, or reduction, of parvovirus. The filter can be of any material known to those of skill in the art to be compatible with the ECM compositions provided herein, such as cellulose or polyethersulfone. The filtration can be repeated as many times as desired by one of skill in the art. Presence of virus can be detected according to standard techniques to monitor clearance.

### 4.3. USES

The ECM compositions provided herein (see Section 4.1) can be used in numerous ways and for many purposes, including, but not limited to, use of the ECM compositions in the manufacture of engineered tissue and organs, including structures such as patches or plugs of tissues or matrix material, prosthetics, and other implants; use of the ECM compositions as tissue scaffolding; use of the ECM compositions in the repair of or dressing of wounds; use of the ECM compositions as hemostatic devices; use of the ECM compositions as devices for use in tissue repair and support, such as sutures, surgical and orthopedic screws, surgical and orthopedic plates, natural coatings or components for synthetic implants, cosmetic implants and supports; use of the ECM compositions in the repair of or as structural support for organs or tissues; use of the ECM compositions for substance delivery; use of the ECM compositions as bioengineering platforms; use of the ECM compositions as platforms for testing the effect of substances upon cells; and use of the ECM compositions in cell culture. Further, the ECM compositions can be used for cosmetic purposes.

In certain embodiments, use of the ECM compositions provided herein requires administration of an ECM composition provided herein to a subject. As used herein, the term "subject" refers to animals, such as mammals, including, but not limited to, primates (e.g., humans), cows, sheep, goats, horses, dogs, cats, rabbits, rats, mice and the like. In a specific embodiment, the subject is a human.

Methods of in vivo administration of the ECM compositions provided herein to a subject include, but are not limited to, oral administration (e.g. buccal or sublingual administration), topical application, aerosol application, transdermal administration, intradermal administration, subdermal administration, intramuscular administration, and surgical administration.

The ECM compositions provided herein can be applied in the form of creams, gels, solutions, suspensions, liposomes, particles, or other means known to one of skill in the art of formulation and delivery of therapeutic and cosmetic compounds. Some examples of appropriate formulations for subcutaneous administration include but are not limited to implants, depot, needles, capsules, and osmotic pumps. Some examples of appropriate formulations for oral administration include but are not limited to: pastes, patches, sheets, liquids, syrups, suspensions, aerosols and mists. Some examples of appropriate formulations for transdermal administration include but are not limited to creams, pastes, patches, sprays, and gels. Some examples of appropriate delivery mechanisms for subcutaneous administration include but are not limited to implants, depots, needles, capsules, and osmotic pumps.

The ECM compositions provided herein can be administered to a subject in any form and/or concentration that will produce desired physiological or pharmacological results. Form and concentration of the ECM composition will depend upon therapeutic endpoint desired, the desired effective concentration at the site of action or in a body fluid, and the type of administration. Information regarding administration of substances to subjects is known to persons of ordinary skill in the art and may be found in references such as L. S. Goodman and A. Gilman, eds, The Pharmacological Basis of Therapeutics, Macmillan Publishing, New York, and Katzung, Basic & Clinical Pharmacology, Appleton & Lang, Norwalk, Conn., (6th Ed. 1995). A clinician skilled in the art of the desired therapy may chose specific form and concentrations, and frequency of administration, as required by the circumstances and the substances to be administered.

### 4.3.1. Use in Treatment of Wounds

The ECM compositions provided herein may be used in the treatment of wounds.

The ECM compositions provided herein may be used to treat a wound by placing the ECM composition directly over the skin of the subject at the site of the wound, so that the wound is covered. The ECM compositions provided herein may be used to treat a wound by using the ECM composition as an implant, *e*.*g*., as a subcutaneous implant. One of skill in the art will recognize that certain formulations of the ECM composition are suitable for such uses. For example, an ECM composition formulated as a sheet can be used to treat a wound, by placing the ECM sheet over the wound on the skin of a subject.

When used in the treatment of a wound, an ECM composition provided herein can be formulated to comprise one or more pharmacologically active agents including, but not limited to, platelet-derived growth factor, insulin-like growth factor, epidermal growth factor, transforming growth factor beta, angiogenesis factor, antibiotics, antifungal agents, spermicidal agents, hormones, enzymes, and enzyme inhibitors.

Wounds that can be treated with the ECM compositions provided herein include, but are not limited to, epidermal wounds, skin wounds, chronic wounds, acute wounds, external wounds, internal wounds (*e*.*g*., an ECM composition may be wrapped around an anastosmosis site during surgery to prevent leakage of blood from suture lines, and to prevent the body from forming adhesions to the suture material), congenital wounds (*e*.*g*., dystrophic epidermolysis bullosa), pressure ulcers (*e*.*g*., decubitus ulcers), partial and full-thickness wounds, venous ulcers, diabetic ulcers, chronic vascular ulcers, tunneled/undermined wounds, surgical wounds (*e*.*g*., donor sites/grafts, post-Moh's surgery, post-laser surgery, podiatric, wound dehiscence), trauma wounds (*e*.*g*., abrasions, lacerations, second-degree burns, and skin tears), and draining wounds.

The ECM compositions provided herein may be used in the treatment of burns and/or conditions associated with burns, including, but not limited to, first-degree burns, second-degree burns (partial thickness burns), third-degree burns (full thickness burns), infections of burn wounds, infection of excised and unexcised burn wounds, loss of epithelium from a previously grafted or healed burn wound, and burn wound impetigo.

### 4.3.2. Dental

The ECM compositions provided herein may be used in the treatment of dental diseases and disorders.

The ECM compositions provided herein may be used in periodontal surgery, guided tissue regeneration for regeneration of periodontal tissue, guided bone regeneration, and/or root coverage. Such methods encompass the use of the ECM compositions to promote regeneration of periodontal intrabony defects, including but not limited to matched bilateral periodontol defects, interdental intrabony defects, deep 3-wall intrabony defects, 2-wall intrabony defects, and intrabony defects 2 and 3.

The ECM compositions provided herein may be used in the treatment of class II furcation defects including but not limited to bilateral defects, paired buccal Class II mandibular molar furcation defects, and bilateral mandibular furcation defects.

The ECM compositions provided herein may be used in the treatment of periodontal disease including but not limited to, periodontitis and gingivitis. An ECM composition provided herein can be used to treat a subject with a periodontal disease by, *e*.*g*., inserting the ECM composition, which can be impregnated with an antibiotic such as chlorhexidine gluconate, into one or more periodontal pockets in the subject, *e.g.,* greater than or equal to 5 mm.

### 4.3.3 Oral Lesions

In certain embodiments, the ECM compositions provided herein are used in the treatment of oral lesions, wherein said lesions are not caused by a dental procedure or by oral surgery. In certain embodiments, provided herein is a method of treating a subject who has an oral lesion comprising administering to the subject, *e*.*g*., administering to the oral lesion, a therapeutically-effective amount of an ECM composition provided herein. In this context, "therapeutically effective amount" means an amount of an ECM composition that acts to reduce or eliminate at least one symptom or aspect of the oral lesion. For example, the ECM composition can be administered in order to repair the lesion, or can be administered as a palliative, *e*.*g*., to reduce pain or inflammation caused by or associated with the oral lesion.

An ECM composition provided herein may be administered directly into an oral lesion as a means of treatment. An ECM composition provided herein may be administered adjacent to or at the periphery of at least a part of the oral lesion. Such administration can be, for example, by placement of a sheet of the ECM composition over at least a portion, or the whole of, the oral lesion. The ECM composition may be administered to the oral lesion as a paste. The ECM composition may be administered to the oral lesion in the form of a spray or aerosol. The ECM composition may be administered to the oral lesion in the form of a solution, *e*.*g*., in a mouthwash.

Oral lesions treated in accordance with the methods provided herein can be caused by any condition or treatment known in the art to cause oral lesions. An may be aft oral lesion treated using an ECM composition provided herein may be caused by or associated with desquamation, *e*.*g*., a desquamating oral disorder. An oral lesion treated using an ECM composition provided herein may be an aphthous ulcer, *e.g.,* an aphthous ulcer caused by, or part of, Behcet's disease. An oral lesion treated using an ECM composition provided herein may be caused by, or is associated with, osteonecrosis of the jaw of a subject.

An oral lesion treated using an ECM composition provided herein may be caused by or is associated with, graft-versus-host disease. An oral lesion treated using an ECM composition provided herein may be caused by or associated with use of melphalan by the subject having the oral lesion.

An oral lesion treated using an ECM composition provided herein may be caused by or associated with chemotherapy, *e*.*g*., chemotherapy that has been administered to the subject to treat a tumor, blood cancer, or other type of cancer. The oral lesion may be caused by post-chemotherapy oral mucositis or chemotherapy-induced oral mucositis. The oral lesion may be, or may be diagnosed as, aphthous stomatitis, *e.g.*, idiopathic aphthous stomatitis. The oral lesion may be caused by or associated with use of an mTOR (mammalian target of rapamycin) inhibitor by the subject having the oral lesion. The oral lesion may be caused by or associated with use of 5-fluorouracil by the subject having the oral lesion. In specific examples, in which the oral lesion is caused by or associated with chemotherapy, *e.g.,* is caused by or associated with use of a chemotherapeutic agent, the chemotherapeutic agent is, *e.g.,* an alkylating agent *(e.g.,* busulfan, cisplatin, carboplatin, cyclophosphamide, dacarbazine, ifosfamide, mechlorethamine or melphalan); an anti-metabolite (*e*.*g*., 5-fluorouracil, methotrexate, gemcitabine, cytarabine, or fludarabine); antibiotics having an antitumor effect (*e*.*g*., bleomycin, dactinomycin, daunorubicin, doxorubicin, or idarubicin); or mitotic inhibitors (*e*.*g*., paclitaxel, docetaxel, etoposide, vinblastine, vincristine or vinorelbine). In another specific example, development of said oral lesion in said subject, wherein said subject is receiving or has received a course of therapy, *e*.*g*., chemotherapy, has caused, or is expected to cause, a premature termination of said course of therapy. In this context, "premature termination" means termination of the course of therapy prior to what has been prescribed for said subject, partially or wholly as a result of said oral lesion.

An oral lesion treated using an ECM composition provided herein may be caused by or associated with administration of an antibody to a subject requiring treatment. The antibody may be an anti-CD20 antibody. The antibody may be rituximab (*e.g.,* RITUXAN^{®}), ofatumumab (*e.g.,* ARZERRA^{®}), veltuzumab or ocrelizumab. The antibody may be an anti-tumor necrosis factor antibody. The antibody may be adalimumab (*e*.*g*., HUMIRA^{®}), etanercept (*e.g.,* ENBREL^{®}), infliximab (*e.g.,* REMICADE^{®}), certolizumab pegol (*e.g.,* CIMZIA^{®}), natalizumab (*e.g.,* TYSABRI^{®}) or golimumab (*e.g.,* SIMPONI^{®}). In another specific example development of said oral lesion in said subject, wherein said subject is receiving or has received a course antibody therapy has caused a premature termination of said course of antibody therapy. In this context, "premature termination" means termination of the course of antibody therapy prior to what has been prescribed for said subject, partially or wholly as a result of said oral lesion.

### 4.3.4 Void Filling

The ECM compositions provided herein may be used to seal, fill, and/or otherwise treat a void within the body of a subject. As used herein, the term "void" is intended to encompass any undesirable hollow space in a subject created by, *e.g.,* aging, disease, surgery, congenital abnormalities, or a combination thereof. For example, a void may be created following the surgical removal of a tumor or other mass from the body of a subject. Non-limiting examples of voids which may be filled with the ECM compositions provided herein include a fissure, fistula, divercula, aneurysm, cyst, lesion, or any other undesirable hollow space in any organ or tissue of a subject's body.

The ECM compositions provided herein may be used to fill, seal and/or otherwise treat, in whole or in part, a crevice, fissure, or fistula within a tissue, organ, or other structure of the body (*e.g.,* a blood vessel), or junctures between adjacent tissues, organs or structures, to prevent the leakage of biological fluids, such as blood, urine, or other biological fluids. For example, the ECM compositions provided herein can be injected, implanted, threaded into, or otherwise administered into fistula between viscera, or into the opening or orifice from a viscus to the exterior of the subject's body. The ECM compositions provided herein can be used to fill a void or other defect formed by these pathological states and stimulate fibroblast infiltration, healing, and ingrowth of tissue.

Described herein is a method to fill, seal, and/or otherwise treat a fistula in a subject, said method comprising injecting or otherwise administering to the subject an ECM composition provided herein. The ECM composition can be administered to the subject by injection through a needle into one of the fistular orifices and filling most or all of the branches of the orifice. Alternatively, strings or rods of the ECM composition can be threaded into the fistulae lesions through an orifice, or the collagen can be introduced into the subject with a catheter. Various types of fistulae can be filled, sealed and/or otherwise treated using the ECM compositions provided herein, such as anal, arteriovenous, bladder, carotid-cavernous, external, gastric, intestinal, parietal, salivary, vaginal, and anorectal fistulae, or a combination thereof.

Described herein is a method to fill, seal and/or otherwise treat a diverticulum in a subject, said method comprising injecting or otherwise administering to the subject an ECM composition provided herein. Diverticulae are abnormal physiological structures that are pouches or sac openings from a tubular or saccular organ, such as the intestine, the bladder, and the like, and can be filled or augmented using the ECM compositions provided herein.

Described herein is a method to fill, seal and/or otherwise treat a cyst in a subject, said method comprising injecting or otherwise administering to the subject an ECM composition provided herein. The cyst may be a pseudocyst, which has an accumulation of, *e.g.,* fluid but does not comprise an epithelial or other membranous lining. Additional non-limiting examples of cysts that can be filled, sealed and/or otherwise treated include sebaceous, dermoid, bone, or serous cysts, or a combination thereof.

The ECM compositions provided herein can be injected or otherwise administered to fill in whole, or in part, any void created as a result of surgical, chemical, or biological removal of unnecessary or undesirable growths, fluids, cells, or tissues from a subject. The ECM composition can be locally injected or otherwise administered at the site of the void so as to augment the remaining and surrounding tissue, aid in the healing process, and minimize the risk of infection. This augmentation is especially useful for void sites created after tumor excision, such as after breast cancer surgery, surgery for removal of tumorous connective tissue, bone tissues or cartilage tissue, and the like.

### 4.3.5 Tissue Bulking

The ECM compositions provided herein can be used for tissue bulking. As used herein, "tissue bulking" refers to any change of the natural state of a subject's (*e*.*g*., a human's) non-dermal soft tissues due to external acts or effects. The tissues encompassed herein include, but not limited to, muscle tissues, connective tissues, fats, and, nerve tissues. The tissues may be part of many organs or body parts including, but not limited to, the sphincter, the bladder sphincter and urethra.

### 4.3.6 Urinary Incontinence

In another embodiment, the ECM compositions provided herein can be used for treatment of urinary incontinence (including stress urinary incontinence), which is the sudden leakage of urine that occurs with activities that result in an increase in intra-abdominal pressure, such as coughing, sneezing, laughing or exercise. In accordance with such embodiments, an ECM composition provided herein can be, *e.g.,* injected into a subject so as to augment the subject's sphincter tissue, thereby improving or restoring in the subject. The ECM composition can be injected or otherwise administered periurethrally to increase tissue bulk around the urethra for the management and/or treatment of urinary incontinence. Improvement in stress incontinence can achieved by increasing the tissue bulk and thereby increasing resistance to the outflow of urine.

The ECM composition may be injected or otherwise administered to a subject in the area around the urethra, for example, to close a hole in the urethra through which urine leaks out or to build up the thickness of the wall of the urethra so it seals tightly when urine is being held back.

The ECM composition may be injected or otherwise administered to a subject around the urethra just outside the muscle of the urethra at the bladder outlet. Injecting the bulking material can be done through the skin, through the urethra, or, in women, through the vagina.

### 4.3.7 Vesicoureteral Reflux

In another embodiment, the ECM compositions provided herein can be used for treatment of vesicoureteral reflux (VUR) (or urinary reflux), which is characterized by the retrograde flow of urine from the bladder to the kidneys. In accordance with such embodiments, an ECM composition provided herein can be injected or otherwise administered to a subject in need thereof, wherein the ureteral wall of the subject is augmented, and the symptoms of VUR are reduced or eliminated. The composition can be injected *(e.g.,* a subtrigonal injection) or otherwise administered, such as under endoscopic guidance, into the detrusor backing under the ureteral orifice using any method known to those in the art.

### 4.3.8 Gastroesophageal Reflux Disease

In another embodiment, the ECM compositions provided herein can be used for treatment of gastroesophageal reflux disease (GERD), which is a disorder that usually occurs because the lower esophageal sphincter (LES)--the muscular valve where the esophagus joins the stomach--does not close properly, relaxes or weakens, and stomach contents leak back, or reflux, into the esophagus. In accordance with such embodiments, an ECM composition provided herein can be injected or otherwise administered to a subject in need thereof, wherein the LES of the subject is augmented, and the symptoms of GERD are reduced or eliminated. The ECM composition may be administered under endoscopic guidance into the esophageal wall at the level of the esophagogastric junction. Intended to impede reflux, the bulking effect results from a combination of the retained material and consequent tissue response. The ECM composition can be injected through standard or large-bore (*e*.*g*., large gauge) injection needles.

### 4.3.9 Vocal Cords and Larynx

In another embodiment, the ECM compositions provided herein can be used in the management or treatment of a disease, disorder (such as a neurological disorder), or other abnormality that affects one or both vocal cords (folds) and/or the larynx (voice box). Non-limiting examples of such diseases, disorders or other abnormalities of the larynx and vocal cords include glottic incompetence, unilateral vocal cord paralysis, bilateral vocal cord paralysis, paralytic dysphonia, nonparalytic dysphonia, spasmodic dysphonia or a combination thereof. The ECM compositions provided herein can be used to manage or treat diseases, disorders or other abnormalities that result in the vocal cords closing improperly, such as an incomplete paralysis of the vocal cord ("paresis"), generally weakened vocal cords, for instance, with old age ("presbylaryngis"), and/or scarring of the vocal cords *(e.g.,* from previous surgery or radiotherapy).

The ECM compositions provided herein can be used to provide support or bulk to a vocal fold in a subject that lacks the bulk (such as in vocal fold bowing or atrophy or the mobility (such as in paralysis) the vocal cord once had. The vocal cords and/or other soft tissues of the larynx can be augmented with the ECM compositions provided herein, either alone or in combination with other treatments or medications. An ECM composition provided herein may augment or add bulk to one (or both) vocal folds so that it can make contact with the other vocal fold.

Any one of a number of procedures well known to one of skill in the art can be used for administration of ECM compositions provided herein to a vocal cord(s) or larynx of a subject. A curved needle may be used to inject an ECM composition provided herein through the mouth of the subject. A needle (such as a higher gauge, short needle) may be used to inject an ECM composition provided herein directly through the skin and the Adam's apple of the subject.

The ECM compositions provided herein can be used in the management or treatment of vocal cord paralysis. The ECM compositions provided herein may be used to manage or treat unilateral or bilateral vocal cord paralysis, or a symptom related thereto in a subject, by injecting or otherwise administering the ECM composition to the subject, wherein vocal fold closure is improved in the subject. The ECM composition may augment or adds bulk to one (or both) paralyzed vocal fold so that it can make contact with the other vocal fold. The injection of ECM composition to the subject can be through the subject's mouth or directly through the skin and Adam's apple.

The ECM compositions provided herein can be used to treat dysphonia, which is any impairment of the voice or difficulty speaking.

### 4.3.10 Glottic Incompetence

In another embodiment, the ECM compositions provided herein can be used for the management or treatment of glottic incompetence. Percutaneous laryngeal collagen augmentation can occur by injection of an ECM composition provided herein into the vocal cords of a subject using methods known in the art. In some cases, the subject has hypophonia and/or glottic incompetence that affects the voice function of the larynx, increased muscle rigidity, and decreased ability for movement of the thyroarytenoid muscle. The hypophonia may be a result of Parkinson's Disease. The ECM composition can be used for the management or treatment of glottic incompetence in a subject in need thereof by injecting or otherwise administering the ECM composition to the vocal cords of the subject, wherein the injection augments the vocal cord and improves glottic closure, such that glottic incompetence is reduced or eliminated in the subject.

### 4.3.11 Bioengineering

The ECM compositions provided herein can be used for bioengineering of tissue or organs, which can be used for, *e.g.,* tissue replacement applications. Examples of bioengineered components that can be generated using the ECM compositions provided herein include, but are not limited to, bone, dental structures, joints, cartilage, skeletal muscle, smooth muscle, cardiac muscle, tendons, menisci, ligaments, blood vessels, stents, heart valves, corneas, ear drums, nerve guides, tissue or organ patches or sealants, a filler for missing tissues, sheets for cosmetic repairs, skin (sheets with cells added to make a skin equivalent), soft tissue structures of the throat such as trachea, epiglottis, and vocal cords, other cartilaginous structures such as nasal cartilage, tarsal plates, tracheal rings, thyroid cartilage, and arytenoid cartilage, connective tissue, vascular grafts and components thereof, and sheets for topical applications, and repair to or replacement of organs such as livers, kidneys, and pancreas.

### 4.3.12 Cosmetic Applications

The ECM compositions provided herein are further useful in cosmetic applications. Generally, such cosmetic uses are based on the fact that the ECM compositions provided herein be used to fill in lines, creases, and other wrinkles in or on the skin of a subject and thus restore a smoother, more youthful-looking appearance.

The ECM compositions provided herein can be used for skin augmentation in a subject. A method for skin augmentation in a subject may comprise injecting or otherwise administering an ECM composition provided herein to an area of the face or body of a subject in need of augmenting, wherein the area of the face or body of the subject is augmented as compared to the area prior to administration of the collagen. "Skin augmentation," as used herein, refers to any change of the natural state of a subject's (*e*.*g*., a human's) skin and related areas due to external acts or effects. Non-limiting areas of the skin that may be changed by skin augmentation include the epidermis, dermis, subcutaneous layer, fat, arrector pill muscle, hair shaft, sweat pore, sebaceous gland, or a combination thereof.

Exemplary cosmetic uses of the ECM compositions provided herein include use of the ECM compositions to augment creased or sunken areas of the face and/or to add or increase the fullness to areas of the face and body of a subject; use of the ECM compositions to treat skin deficiencies including, but not limited to, wrinkles, depressions or other creases (*e*.*g*., frown lines, worry lines, crow's feet, marionette lines), stretch marks, internal and external scars (such as scars resulting from injury, wounds, accidents, bites, or surgery); use of the ECM compositions to correct "hollow" eyes and visible vessels resulting in dark circles around the eyes; use of the ECM compositions to correct or supplement plastic surgery, including correction of the undereye after aggressive removal of undereye fat pads from lower blepharoplasty, correction of the lower cheek after aggressive buccal fat extraction, and correction of the results of rhinoplasty, skin graft or other surgically-induced irregularities, such as indentations resulting from liposuction; use of the ECM compositions to correct facial or body scars (*e*.*g*., wound, chicken pox, or acne scars); and use of the ECM compositions for facial reshaping.

### 5. HITS

Described herein, but not explicitly claimed, are kits comprising the ECM compositions provided herein.

In one example, provided herein is a kit comprising an ECM composition provided herein, wherein said ECM composition is formulated as a sheet. The ECM sheet is provided in sterile form, and packaged in a pouch. The sheets of ECM provided in such kits can be of varying size (*e.g.,* 5 x 5 cm or 9 x 9 cm) and thickness (*e.g.,* 1.5-2.5 mm) and are ready for use by a practitioner, *e*.*g*., use as a wound dressing.

In another example, provided herein is a kit comprising an ECM composition provided herein, wherein said ECM composition can be formulated as a flowable matrix. In such kits, an ECM composition is provided in sterile form, and packaged in a glass vial, as micronized ECM. Such vials can comprise varying amounts of micronized ECM, *e.g.,* 200 mg of micronized ECM. Kits comprising an ECM composition that is to be formulated as a flowable matrix may further comprise components suitable for use in suspension of the micronized ECM, such as a suspension solution (*e*.*g*., saline) and a syringe with a needle and flexible applicator.

In another example, provided herein is a kit comprising an ECM composition provided herein, wherein said ECM composition is formulated as a particulate. In such kits, an ECM composition is provided in sterile form, and packaged in a glass vial, as milled ECM. Such vials can comprise varying amounts of milled ECM, *e.g.,* 100 mg or 200 mg of milled ECM.

The kits provided herein can comprise a label or labeling with instructions on using the ECM composition provided in the kit. The kits can comprise components useful for carrying out methods for which the ECM compositions are useful, such as means for administering the ECM composition in the kit, *e.g.,* one or more spray bottles, tweezers, a spatula (for applying paste or particulate), cannulas, catheters, etc.

### 6. EXAMPLES

### 6.1. Example 1: Method of Producing Placental ECM

This example describes methods of producing placental ECM, initially formulated as a paste.

Method 1: A previously isolated, frozen human placenta was obtained and allowed to thaw at room temperature for ~24 hours. After the placenta was thawed, the amnion, chorion, and umbilical cord were removed from the placenta and discarded. Next, the placenta was cut into 2 × 2 centimeter strips for processing.

The placental tissue then was placed in a receptacle containing 1.5 liters of a 1 M NaCl solution, and homogenized using an Omni Mixer Homogenizer (Omni International, Kennesaw, GA)). Next, the homogenized placental tissue was placed in a processing bag, and the bag was filled with a 1 M NaCL solution to a total volume of 9.2 liters. The homogenized placental tissue then was washed three times in a 1 M NaCl solution as follows: (i) the processing bag was agitated on an orbital shaker for 10 minutes, (ii) the placental tissue was allowed to settle in the processing bag for 10 minutes, and (iii) 6.2 liters of the supernatant was removed from the processing bag by gravity drainage, a step that removes blood and debris from the mixture.

After the third washing step, the washed placental tissue was allowed to mix in the 1 M NaCl solution (3 liters total of mixture) on an orbital shaker for ~18-26 hours at room temperature. Next, the placental tissue was washed four times with sterile water, in the same manner described above for the NaCl washes. After the fourth wash in water, the placental tissue was allowed to mix in the water (3 liters total of mixture) on an orbital shaker for ∼18-26 hours at room temperature. After the -18-26 hour mixing in water, the placental tissue was washed a final time with water, as described above, then 6.2 L of 3% sodium deoxycholate was added to the mixture, for a final concentration of 2% sodium deoxycholate in the mixture.

The placental tissue was allowed to mix in the 2% sodium deoxycholate solution on an orbital shaker for -72 hours at room temperature. After the -72 hour mixing, the placental tissue was washed with sterile water five times, in the manner described above. After the final addition of water, the pH of the solution was brought to about 10-12 by dropwise addition of 1M NaOH, resulting in a basic solution. The placental tissue was allowed to mix in the basic solution on an orbital shaker for -30 minutes at room temperature. After the ~30 minutes of mixing, the pH of the solution was adjusted to about 7.0-7.5 by dropwise addition of 0.1 N HCl.

The supernatant then was removed from the processing bag and the placental tissue remaining was collected and centrifuged. After centrifugation, the supernatant was removed and the collected placental tissue was resuspended in sterile water, as a final wash step, and centrifuged again, followed by discarding of the supernatant. The resulting composition represented placental ECM comprising collagen and elastin, and was in the form of a white paste.

Method 2: Upon being released for processing, a frozen human placenta was thawed at 2 - 8° C and then transferred to a biological safety cabinet (BSC), and then processed as done in Method 1. The placenta was removed from its storage container and placed on a sterile disposable tray. The placenta was then cleaned to remove excess blood and blood clots and then cut into small segments. The cut placental material was suspended in sterile water and then homogenized using a mechanical homogenizer, which generated small tissue particulates with increased surface area, allowing for more effective separation and removal of cells and cellular debris from placental ECM. The homogenized tissue from the placenta was transferred into a sterile processing bag with sterile 1 M sodium chloride solution. The tissue was washed several times with sterile 1 M sodium chloride (NaCl) by shaking on an orbital shaker; the NaCl solution was exchanged by allowing the placental tissue to settle, followed by draining and refreshing with additional sterile 1 M NaCl solution. The placental tissue was held for 18-24 hours with shaking in sterile 1 M NaCl solution, followed by repeated washing with sterile water. All processing steps were conducted at room temperature. The exposure of the placental tissue to a high concentration of sodium chloride, followed by water constitutes an "osmotic shock" to the tissue, which serves to clean the tissue of blood, blood components, cells and cellular debris. The placental tissue was subjected to a second "osmotic shock" before the next step, a detergent wash.

The rinsed placental tissue was held for 48-72 hours with sterile 0.1-0.3 % sodium deoxycholate (DOC) solution and 4-8 mM ethylenediaminetetraacetic acid (EDTA) solution with shaking in the bio-processing bag at room temperature. Following a sterile water rinse, the tissue was subjected to a second wash with DOC/EDTA for 18-24 hours. Sterile water was then used to rinse the tissue and remove the DOC and EDTA.

Upon completion of the water wash, the supernatant was removed from the bio-processing bag and replaced with a solution of 2 mM magnesium chloride and 10 U/mL of BENZONASE^{®}, pH 8-9, and mixed for 18-24 hours at room temperature. BENZONASE^{®} is an endonuclease that degrades all forms of nucleic acids (RNA & DNA); the resulting shorter polynucleotide fragments are washed out with sequential rinses of the placental tissue.

After rinsing of the placental tissue to remove residual nucleic acids, the material was subjected to low and high pH treatments as viral inactivation steps. In the first step, the placental tissue was subjected to a pH of 3.3 or less in the presence of sterile 0.67 M NaCl solution and allowed to shake on an orbital shaker for 24 hours at 22 +/- 1°C. In the second step, the pH of the solution was adjusted to ≥13 using sodium hydroxide (NaOH) and allowed to mix in the bio-processing bag, on an orbital shaker for a minimum of 4 hours at 22 +/- 1° C. At the end of NaOH exposure, the pH of the solution was adjusted to a range of 5.5-9.0.

Upon completion of the acid and base treatments, the tissue was incubated with 1M NaCl and allowed to mix on an orbital shaker for 48-72 hours. Following the NaCl treatment, the placental ECM was washed with sterile water for 18-24 hrs to remove debris and residual contaminants. ECM paste was generated by centrifuging the suspension. The ECM paste was stored in a -20° C freezer until the product was formulated and sterilized.

Formulation as sheet: To generate ECM sheets, the ECM paste was thawed at 2°C to 22°C for 24-48 hours, and resuspended in sterile phosphate buffer in a biological safety cabinet. The tissue was homogenized to prepare a homogenous ECM suspension, which was distributed into sterile plastic molds and frozen. The frozen molds of ECM were dehydrated using lyophilization. The resulting dehydrated ECM wafers were re-hydrated with sterile water and then compacted on a vacuum-assisted dryer for 18-36 hours at room temperature. Sheets were placed into a double pouch and sealed using a medical grade sealer, labeled, and sterilized using gamma radiation.

Formulation as a particulate: To generate ECM particulate, the ECM paste is resuspended in sterile water, transferred into molds, frozen using a Controlled Rate Freezer (Thermo Scientific, Marietta, OH) and lyophilized in a freeze-dryer (LabConco, Kansas City, MO) for 48 hours. The lyophilized ECM is milled using a jet mill (Fluid Energy, Telford, PA), resulting in ECM particulate. The milled ECM powder can be filled into amber glass vials and sealed.

Formulation as flowable matrix: ECM flowable matrix can be prepared using ECM particulate. Generally, ECM flowable matrix can be prepared by suspending lyophilized, milled ECM (ECM particulate) in, *e.g.,* sterile water or saline solution.

### 6.2. Example 2: Determination of Collagen Content of ECM Composition

This Example explains the analysis of collagen content of the ECM composition.

ECM composition, prepared as described in Example 1 in particulate, sheet or flowable matrix form, was analyzed for total collagen content using a colorimetric hydroxyproline assay. Collagen is a unique protein in that it has a very high proline concentration. In addition, collagen is post-translationally modified to hydroxyproline. As such, quantification of hydroxyproline, which can be obtained from collagen hydrolyzed in hot hydrochloric acid can be used as a surrogate for quantification of collagen.

ECM samples were hydrolyzed using 6 N HCl at 110° C, and hydroxyproline was then oxidized to pyrrole-2-carboxylic acid (pyrrole) using Chloramine-T (N-chloro 4-methylbenzenesulfonamide). Color was developed using 4-Dimethylaminobenzaldehyde (DMAB), and hydroxyproline content determined by reading the absorbance at 550 nm. Sample absorbances were interpolated against a hydroxyproline standard curve using known amounts of hydroxyproline.

The ECM composition was determined to comprise a range of 31% total collagen by weight to 53% total collagen by weight of the ECM composition (Table 1). 14/16 samples fell within a range of 34% to 43% total collagen by weight of the ECM composition, and 12/16 fell within a range of 37% to 42% total collagen by weight of the ECM composition. In contrast, comparator matrix products derived from porcine urinary bladder matrix (MATRISTEM^{®} Wound Matrix sheets or MATRISTEM MICROMATRIX^{®} particulates) and fetal bovine dermis matrix (PRIMATRIX^{™} Dermal Repair Matrix) were found to comprise 67%, 67%, and 69% total collagen by weight, respectively.

**Table 1: Total collagen as a percent by dry weight of ECM composition**

| **Sample** | **Type** | **Collagen % by weight** |
|---|---|---|
| 1 | Particulate | 37 |
| 2 | Sheet | 43 |
| 3 | Particulate | 42 |
| 4 | Particulate | 34 |
| 5 | Sheet | 41 |
| 6 | Particulate | 38 |
| 7 | Particulate | 38 |
| 8 | Flowable Matrix | 42 |
| 9 | Sheet | 38 |
| 10 | Particulate | 53 |
| 11 | Particulate | 40 |
| 12 | Particulate | 42 |
| 13 | Particulate | 38 |
| 14 | Particulate | 37 |
| 15 | Particulate | 42 |
| 16 | Particulate | 31 |
| Porcine urinary bladder matrix | Sheet | 67 |
| Porcine urinary bladder matrix | Particulate | 67 |
| Fetal bovine dermal matrix | Sheet | 69 |

### 6.3. Example 3: Determination of Elastin Content of ECM Composition

This Example explains the analysis of elastin content of the ECM composition.

ECM composition, prepared as described in Example 1 as either particulate or sheets, was analyzed for elastin content using the Fastin Elastin Assay kit (BioColor, Ltd. (UK)), which uses 5, 10, 15, 20-tetraphenyl-21, 23-porphine tetra-sulfonate (TPPS) as a dye to stain extracted and solubilized α-elastin from test samples. Elastin was extracted from ECM composition samples in 0.25 M oxalic acid at 100° C. Elastin was then precipitated with trichloroacetic acid and hydrochloric acid (TCA/HCl), and then stained with TPPS. The TPPS was then dissociated from the elastin, and released TPPS levels were quantified by spectrophotometry. Elastin levels in the ECM composition samples were determined by interpolation against an elastin standard curve.

The ECM composition was determined to have a range of 13% to 29% elastin by weight of the ECM composition (Table 2). 14/16 samples fell within a range of 16% to 24% elastin by weight of the ECM composition, 13/16 samples fell within a range of 17% to 24% elastin by weight of the ECM composition, and 10/16 samples fell within a range of 20% to 24% elastin by weight of the ECM composition. In contrast, the comparator matrix products derived from porcine urinary bladder matrix (MATRISTEM^{®} Wound Matrix sheets or MATRISTEM MICROMATRIX^{®} particulates) were found to comprise 4% elastin. Elastin was not detected in fetal bovine dermis matrix (PRIMATRIX^{™} Dermal Repair Matrix).

In Tables 2 and 3, sample 8 represents the same ECM composition as sample 8 in Table 1. However, the type of ECM composition analyzed differed (*i.e.,* the flowable matrix form of the ECM composition was analyzed in Table 1, whereas the particulate form of the ECM composition was analyzed in tables 2 and 3).

**Table 2: Elastin percentage of total ECM composition by dry weight**

| **Sample** | **Type** | **Elastin % by Weight** |
|---|---|---|
| 1 | Particulate | 17 |
| 2 | Sheet | 20 |
| 3 | Particulate | 17 |
| 4 | Particulate | 16 |
| 5 | Sheet | 24 |
| 6 | Particulate | 24 |
| 7 | Particulate | 20 |
| 8 | Particulate | 21 |
| 9 | Sheet | 22 |
| 10 | Particulate | 13 |
| 11 | Particulate | 17 |
| 12 | Particulate | 22 |
| 13 | Particulate | 20 |
| 14 | Particulate | 29 |
| 15 | Particulate | 25 |
| 16 | Particulate | 23 |
| Average: | | 21 |
| MatriStem Wound Matrix | Sheet | 4 |
| Matristem Micromatrix | Particulate | 4 |
| Primatrix Sheet | Sheet | Not detected |

### 6.4. Example 4: Determination of Fibronectin Content of ECM Composition

This Example describes the analysis of fibronectin content of the ECM composition.

ECM composition, prepared as described in Example 1 as either particulate or sheets, was analyzed for fibronectin content using the TaKaRa Fibronectin EIA kit (Mountain View, CA), which is based on a sandwich ELISA method that utilizes two mouse monoclonal anti-human fibronectin antibodies to detect fibronectin by a two-step procedure. Additionally, a specific extraction method for fibronectin using 2M urea buffer was developed.

15 mg of ECM composition was mixed with 3 mL of extraction buffer (2 M urea, 0.05 M PO4, 2 mM PMSF, pH 7.1) and mixed on a stir plate for 4 hours. The sample was then centrifuged for 20 minutes at 10,000 X g. Supernatants were stored at -20°C until the time of analysis, or used immediately. Analysis of 16 ECM composition samples using the TaKaRa Fibronectin EAI Kit was performed according to manufacturer's instructions. A standard curve of fibronectin concentrations was constructed using commercially-available lyophilized fibronectin, and sample fibronectin concentrations were interpolated from the curve.

Fibronectin content of the ECM composition was low, with an average of 197 ng of fibronectin per mg of ECM composition (0.0197% by weight of the ECM composition), and a range of 21.3 ng/mg to 361.3 ng/mg. Samples of porcine urinary bladder matrix (MATRISTEM^{®} Wound Matrix sheets or MATRISTEM MICROMATRIX^{®} particulates) and fetal bovine dermis matrix (PRIMATRIX^{™} Dermal Repair Matrix) were tested but revealed no fibronectin content in the assay, possibly because the antibodies used in the assay did not cross-react with the bovine or porcine matrix.

**Table 3: Fibronectin percentage of total ECM composition by dry weight**

| **Sample** | **Type** | **Average ng/mg** |
|---|---|---|
| 1 | Particulate | 332.7 |
| 2 | Sheet | 181.0 |
| 3 | Particulate | 114.8 |
| 4 | Particulate | 270.0 |
| 5 | Sheet | 21.3 |
| 6 | Particulate | 194.1 |
| 7 | Particulate | 112.6 |
| 8 | Particulate | 150.1 |
| 9 | Sheet | 71.8 |
| 10 | Particulate | 138.1 |
| 11 | Particulate | 97.0 |
| 12 | Particulate | 200.7 |
| 13 | Particulate | 217.0 |
| 14 | Particulate | 361.3 |
| 15 | Particulate | 342.8 |
| 16 | Particulate | 346.8 |
| | **Average** | **197.0** |
| MatriStem | Particulate | Not detected |
| MicroMatrix | | |
| MatriStem | Sheet | Not detected |
| Wound Matrix | | |
| PriMatrix | Sheet | Not detected |

### 6.5. Example 5: Determination of Laminin Composition of ECM Composition

Sixteen samples of ECM composition, prepared as described in Example 1 and formulated as a sheet or particulate, were analyzed for laminin content using the TaKaRa Laminin EIA kit (Mountain View, CA), according to manufacturer instructions. The kit is based on a sandwich ELISA method that utilizes two mouse monoclonal anti-human laminin antibodies to detect laminin by a two-step procedure. Additionally, a specific extraction method for laminin using 2M urea buffer was developed. A standard curve of laminin concentrations was constructed using commercially-available lyophilized laminin, and sample laminin concentrations were interpolated from the curve.

Results: Laminin was not detected in any of the 16 ECM samples. Laminin also was not detected in any of the porcine urinary bladder matrix (sheet or particulate) or fetal bovine dermis sheets analyzed for comparison.

### 6.6. Example 6: Biocompatibility of ECM Composition

ECM composition, prepared as described in Example 1 and formulated as a sheet, a particulate, or a flowable matrix, was tested for biocompatibility in albino New Zealand White Rabbits, 4.7-6.3 months in age and weighing 2.4-2.9 kg at study start. As controls, ECM sheet was compared to MATRISTEM^{®} Wound Matrix (porcine urinary bladder matrix); ECM particulate was compared to MATRISTEM MICROMATRIX^{®}, and ECM flowable matrix was compared to INTEGRA^{™} Flowable Wound Matrix (collagen and glycosaminoglycan).

| Test Article | Amount Used/Site |
|---|---|
| ECM Particulate | 5.0-5.4 mg |
| ECM Flowable | 50 µL |
| ECM Sheet | 1 x 3 x 10 mm pieces |
| MATRISTEM^{®} Particulate | 5.0-5.4 mg |
| Integra Flowable | 50 µL |
| MatriStem Sheet | 1 x 3 x 10 mm pieces |

Preparation of flowable matrix: ECM Flowable and INTEGRA^{™} Flowable were prepared by aspirating over 2 mL of injectable water with a 3 mL Vial Access syringe, eliminating any air bubbles, and adjusting the volume to 2 mL. The cannula was then removed from the Vial Access syringe. This syringe was then connected to an ECM syringe containing 2 mL water tip-to-tip. The water was slowly injected from the vial access syringe into the Vial Access syringe, and carefully mixed using 10 to 15 back and forth movements until the powder was homogenously hydrated. The entire reconstituted paste was pushed into one of the syringes. The empty syringe was then discarded, an empty 1 mL syringe was connected to the remaining ECM paste syringe, and 1 mL of the ECM Flowable or INTEGRA^{™} Flowable paste was transferred into the 1 mL syringe. This last step was repeated with a second 1 mL syringe. The pastes were re-mixed prior to loading into the 1 mL syringe for each dose. INTEGRA^{™} Flowable was mixed with 3.0 mL of saline for injection. The test and control articles were applied directly using the filled 1 mL syringes.

Implantation: For each animal, a 2-4 cm skin incision was made over the midline of the back, extending through the fascia of both paravertebral muscles. For each implant site, a 5 mm incision was made into the paravertebral muscle and a small pocket was generated with hemostats for implantation, approximately 2 cm from the midline and parallel to the muscle fiber axis, allowing at least 2.5 cm between implant sites. Four test or four control articles were implanted in one side of the paravertebral muscle. The test article was implanted on the right side and the control article on the left side for each group. All implant sites were then closed with non-absorbable suture, and the skin incision was closed with suture and/or skin staple. 50 µL of the flowable formulation, 5 mg of particulate formulation and 1 piece (1 x 3 x 10 mm) of sheet formulation were implanted into the generated pocket of the muscle. Control articles were 50 µL of bovine derived wound matrix product (INTEGRA^{™} Flowable), 5 mg of particulate formulation of extra cellular matrix derived from porcine urinary bladder (MATRISTEM MICROMATRIX^{®}), and a sheet formulation (1 x 3 x 10 mm) derived from porcine urinary bladder extra cellular matrix (MATRISTEM WOUND MATRIX^{®}) Animals were killed at weeks 1, 2 or 4 post-implantation, and histology was performed on the

Results: ECM sheet showed demonstrably less tissue reactivity than did the porcine urinary bladder matrix. At week 1 postimplantation, the tissue adjacent to the urinary bladder matrix (UBM) sheet showed distinct signs of granulation and inflammatory response (Fig. 1A), while the ECM sheet showed muscle tissue interspersed with slight infiltration of granulocytes. At weeks 2 and 4, granulation was still in evidence adjacent to the UBM sheet (Figs. 1C and 1E), while tissue adjacent to the ECM sheet showed virtually no granulation and appeared to be normal (Figs. 1D and 1F). The ECM sheet was deemed to be a non-irritant at weeks 1, 2 and 4 post-implantation.

ECM particulate and flowable matrix also engendered less tissue reactivity than the control particulate or flowable matrix (FIGS. 2 and 3). For example, at week 1 postimplantation, the ECM particulate showed some granulation indicating inflammation (FIG. 2B), but significantly less than the UBM particulate (FIG. 2A), while at weeks 2 and 4, the ECM particulate showed a significant reduction of granulation (FIGS. 2D and 2F, respectively) as compared to the UBM particulate, which still showed substantial inflammation at weeks 2 and 4 (FIGS. 2C and 2E, respectively). Similarly, while the bovine derived wound matrix product (INTEGRA^{™} Flowable) showed granulation at week 1 (FIG. 3A), followed by scarring at weeks 2 and 4 (lighter areas in FIGS. 3C and 3E), the ECM flowable showed an inflammatory response substantially only in the first week (FIG. 3B), followed by near-complete healing at weeks 2 and 4 (FIGS. 3D and 3F, respectively).

### 6.7. Example 7: Method of Producing Placental ECM From Placental Chorion

This example describes methods of producing placental ECM from placental chorion, initially formulated as a paste.

Method 1: A previously isolated, frozen human placenta is obtained and allowed to thaw at room temperature for ~24 hours. After the placenta is thawed, the chorion is obtained from the placenta. Next, the placenta is cut into 2 x 2 centimeter strips for processing.

The placental chorion tissue then is placed in a receptacle containing 1.5 liters of a 1 M NaCl solution, and homogenized using an Omni Mixer Homogenizer (Omni International, Kennesaw, GA)). Next, the homogenized placental chorion tissue is placed in a processing bag, and the bag is filled with a 1 M NaCL solution to a total volume of 9.2 liters. The homogenized placental chorion tissue then is washed three times in a 1 M NaCl solution as follows: (i) the processing bag is agitated on an orbital shaker for 10 minutes, (ii) the placental chorion tissue is allowed to settle in the processing bag for 10 minutes, and (iii) 6.2 liters of the supernatant is removed from the processing bag by gravity drainage, a step that removes blood and debris from the mixture.

After the third washing step, the washed placental chorion tissue is allowed to mix in the 1 M NaCl solution (3 liters total of mixture) on an orbital shaker for ∼18-26 hours at room temperature. Next, the placental tissue is washed four times with sterile water, in the same manner described above for the NaCl washes. After the fourth wash in water, the placental chorion tissue is allowed to mix in the water (3 liters total of mixture) on an orbital shaker for -18-26 hours at room temperature. After the -18-26 hour mixing in water, the placental chorion tissue is washed a final time with water, as described above, then 6.2 L of 3% sodium deoxycholate is added to the mixture, for a final concentration of 2% sodium deoxycholate in the mixture.

The placental chorion tissue is allowed to mix in the 2% sodium deoxycholate solution on an orbital shaker for ~72 hours at room temperature. After the ~72 hour mixing, the placental chorion tissue is washed with sterile water five times, in the manner described above. After the final addition of water, the pH of the solution is brought to about 10-12 by dropwise addition of 1M NaOH, resulting in a basic solution. The placental chorion tissue is allowed to mix in the basic solution on an orbital shaker for -30 minutes at room temperature. After the ~30 minutes of mixing, the pH of the solution is adjusted to about 7.0-7.5 by dropwise addition of 0.1 N HCl.

The supernatant then is removed from the processing bag and the placental chorion tissue remaining is collected and centrifuged. After centrifugation, the supernatant is removed and the collected placental chorion tissue is resuspended in sterile water, as a final wash step, and centrifuged again, followed by discarding of the supernatant. The resulting composition represents placental ECM comprising collagen and elastin, and will be in the form of a white paste.

Method 2: Upon being released for processing, a frozen human placenta is thawed at 2 - 8° C and then transferred to a biological safety cabinet (BSC). The placenta is removed from its storage container and placed on a sterile disposable tray. The placenta is then cleaned to remove excess blood and blood clots. The chorion of the placenta is obtained and cut into small segments. The cut placental chorion material is suspended in sterile water and then homogenized using a mechanical homogenizer, which will generate small tissue particulates with increased surface area, allowing for more effective separation and removal of cells and cellular debris from placental ECM. The homogenized tissue from the placental chorion is transferred into a sterile processing bag with sterile 1 M sodium chloride solution. The tissue is washed several times with sterile 1 M sodium chloride (NaCl) by shaking on an orbital shaker; the NaCl solution is exchanged by allowing the placental tissue to settle, followed by draining and refreshing with additional sterile 1 M NaCl solution. The placental tissue is held for 18-24 hours with shaking in sterile 1 M NaCl solution, followed by repeated washing with sterile water. All processing steps are conducted at room temperature. The exposure of the placental chorion tissue to a high concentration of sodium chloride, followed by water constitutes an "osmotic shock" to the tissue, which serves to clean the tissue of blood, blood components, cells and cellular debris. The placental tissue is subjected to a second "osmotic shock" before the next step, a detergent wash.

The rinsed placental chorion tissue is held for 48-72 hours with sterile 0.1-0.3 % sodium deoxycholate (DOC) solution and 4-8 mM ethylenediaminetetraacetic acid (EDTA) solution with shaking in the bio-processing bag at room temperature. Following a sterile water rinse, the tissue is subjected to a second wash with DOC/EDTA for 18-24 hours. Sterile water is then used to rinse the tissue and remove the DOC and EDTA.

Upon completion of the water wash, the supernatant is removed from the bio-processing bag and replaced with a solution of 2 mM magnesium chloride and 10 U/mL of BENZONASE^{®}, pH 8-9, and mixed for 18-24 hours at room temperature. BENZONASE^{®} is an endonuclease that degrades all forms of nucleic acids (RNA & DNA); the resulting shorter polynucleotide fragments are washed out with sequential rinses of the placental tissue.

After rinsing of the placental chorion tissue to remove residual nucleic acids, the material is subjected to low and high pH treatments as viral inactivation steps. In the first step, the placental chorion tissue is subjected to a pH of 3.3 or less in the presence of sterile 0.67 M NaCl solution and allowed to shake on an orbital shaker for 24 hours at 22 +/- 1°C. In the second step, the pH of the solution is adjusted to ≥13 using sodium hydroxide (NaOH) and allowed to mix in the bio-processing bag, on an orbital shaker for a minimum of 4 hours at 22 +/-1° C. At the end of NaOH exposure, the pH of the solution is adjusted to a range of 5.5-9.0.

Upon completion of the acid and base treatments, the tissue is incubated with 1M NaCl and allowed to mix on an orbital shaker for 48-72 hours. Following the NaCl treatment, the placental chorion ECM is washed with sterile water for 18-24 hrs to remove debris and residual contaminants. ECM paste is generated by centrifuging the suspension. The ECM paste can be stored in a -20° C freezer until the product is formulated and sterilized.

Formulation as sheet: To generate ECM sheets, the ECM paste is thawed at 2°C to 22°C for 24-48 hours, and resuspended in sterile phosphate buffer in a biological safety cabinet. The tissue is homogenized to prepare a homogenous ECM suspension, which is distributed into sterile plastic molds and frozen. The frozen molds of ECM are dehydrated using lyophilization. The resulting dehydrated ECM wafers are re-hydrated with sterile water and then compacted on a vacuum-assisted dryer for 18-36 hours at room temperature. Sheets are placed into a double pouch and sealed using a medical grade sealer, labeled, and sterilized using gamma radiation.

Formulation as a particulate: To generate ECM particulate, the ECM paste is resuspended in sterile water, transferred into molds, frozen using a Controlled Rate Freezer (Thermo Scientific, Marietta, OH) and lyophilized in a freeze-dryer (LabConco, Kansas City, MO) for 48 hours. The lyophilized ECM is milled using a jet mill (Fluid Energy, Telford, PA), resulting in ECM particulate. The milled ECM powder can be filled into amber glass vials and sealed.

Formulation as flowable matrix: ECM flowable matrix can be prepared using ECM particulate. Generally, ECM flowable matrix can be prepared by suspending lyophilized, milled ECM (ECM particulate) in, *e.g.,* sterile water or saline solution.

The collagen, elastin, fibronectin, and/or laminin content of the ECM can be analyzed in the manner described in Examples 2, 3, 4, and 5, respectively. Further, biocompatibility of the ECM can be assessed in the manner described in Example 6.

### 6.8. Example 8: Method of Producing Placental ECM From Placental Chorion (2)

This example describes a method for producing a placental ECM composition from placental chorion, initially formulated as a paste.

A human placenta obtained from a mother immediately after a full-term birth, or a previously isolated frozen human placenta that has been allowed to thaw, is utilized. The placenta is washed in 0.5M NaCl. The amnion, umbilical cord and decidua parietalis is removed from the placenta, and the chorionic plate is retained, which is then scraped and cleaned. The scraped, cleaned chorion is rinsed in 0.5 M NaCl and water, and then rinsed overnight in 2% deoxycholic acid, followed by several water rinses. The treated chorion is then ground and freeze dried. The resulting composition is a decellularized ECM paste suitable for further formulation, *e*.*g*., milling and formulation.

Formulation as sheet: To generate ECM sheets, the ECM paste is thawed at 2°C to 22°C for 24-48 hours, and resuspended in sterile phosphate buffer in a biological safety cabinet. The tissue is homogenized to prepare a homogenous ECM suspension, which is distributed into sterile plastic molds and frozen. The frozen molds of ECM are dehydrated using lyophilization. The resulting dehydrated ECM wafers are re-hydrated with sterile water and then compacted on a vacuum-assisted dryer for 18-36 hours at room temperature. Sheets are placed into a double pouch and sealed using a medical grade sealer, labeled, and sterilized using gamma radiation.

Formulation as a particulate: To generate ECM particulate, the ECM paste is resuspended in sterile water, transferred into molds, frozen using a Controlled Rate Freezer (Thermo Scientific, Marietta, OH) and lyophilized in a freeze-dryer (LabConco, Kansas City, MO) for 48 hours. The lyophilized ECM is milled using a jet mill (Fluid Energy, Telford, PA), resulting in ECM particulate. The milled ECM powder can be filled into amber glass vials and sealed.

Formulation as flowable matrix: ECM flowable matrix can be prepared using ECM particulate. Generally, ECM flowable matrix can be prepared by suspending lyophilized, milled ECM (ECM particulate) in, *e.g.,* sterile water or saline solution.

The collagen, elastin, fibronectin, and/or laminin content of the ECM can be analyzed in the manner described in Examples 2, 3, 4, and 5, respectively. Further, biocompatibility of the ECM can be assessed in the manner described in Example 6.

### 6.9. Example 9: Method of Producing Placental ECM From Placental Chorion (3)

This example describes a method for producing a placental ECM composition from placental chorion.

A human placenta obtained from a mother immediately after a full-term birth is utilized. The amnion, umbilical cord and decidua parietalis is removed from the placenta, and the chorionic plate is retained, which is then scraped and cleaned. The scraped, cleaned chorion is rinsed in 1.0 M NaCl and water, and then rinsed overnight in a solution containing 0.067% deoxycholic acid and 4mM EDTA, followed by several water rinses. The chorion is then rinsed overnight in a solution containing 0.39% deoxycholic acid and 8mM EDTA, followed by several water rinses. The treated whole chorion is then freeze dried.

Formulation as sheet: To generate ECM sheets, the ECM is thawed at 2°C to 22°C for 24-48 hours, and resuspended in sterile phosphate buffer in a biological safety cabinet to form an ECM paste. The tissue is homogenized to prepare a homogenous ECM suspension, which is distributed into sterile plastic molds and frozen. The frozen molds of ECM are dehydrated using lyophilization. The resulting dehydrated ECM wafers are re-hydrated with sterile water and then compacted on a vacuum-assisted dryer for 18-36 hours at room temperature. Sheets are placed into a double pouch and sealed using a medical grade sealer, labeled, and sterilized using gamma radiation.

Formulation as a particulate: To generate ECM particulate, the ECM is resuspended in sterile water to form an ECM paste, transferred into molds, frozen using a Controlled Rate Freezer (Thermo Scientific, Marietta, OH) and lyophilized in a freeze-dryer (LabConco, Kansas City, MO) for 48 hours. The lyophilized ECM is milled using a jet mill (Fluid Energy, Telford, PA), resulting in ECM particulate. The milled ECM powder can be filled into amber glass vials and sealed.

Formulation as flowable matrix: ECM flowable matrix can be prepared using ECM particulate. Generally, ECM flowable matrix can be prepared by suspending lyophilized, milled ECM (ECM particulate) in, *e.g.,* sterile water or saline solution.

The collagen, elastin, fibronectin, and/or laminin content of the ECM can be analyzed in the manner described in Examples 2, 3, 4, and 5, respectively. Further, biocompatibility of the ECM can be assessed in the manner described in Example 6.

Characterization of ECM compositions, pre-sterilization, produced using such a method exhibited the dry weight characteristics summarized in Table 4, below:

**Table 4**

| | |
|---|---|
| Collagen | 43-68% |
| Elastin | 18-21% |
| Fibronectin | 27-5322 ng/mg (<0.05%) |
| Laminin | 28-568 ng/ml (<0.05%) |
| Glycosaminoglycans | 0.2 - 1.2 µg/mg (<0.05%) |
| Cytokines | <80 pg/mL (below level of detection) |
| Growth Factors | <80 pg/mL (below level of detection) |
| Deoxycholic Acid | <300 parts per million (below level of detection) |
| Cell-free | >90% |
| Cellular debris-free | >90% |

## Claims

1. An extracellular matrix (ECM) composition comprising:
(a) 35-55% collagen by dry weight and 10-30% elastin by dry weight, or
(b) 40-70% collagen by dry weight and 15-25% elastin by dry weight.

2. The composition of claim 1, wherein said composition comprises:
(a) less than 0.1% fibronectin by dry weight; and/or
(b) an undetectable amount of laminin, or less than about 0.1% laminin by dry weight; and/or
(c) an undetectable amount of glycosaminoglycans, or less than about 0.1% glycosaminoglycans by dry weight; and/or
(d) an undetectable amount of cytokines, growth factors and/or deoxycholic acid.

3. The composition of claim 1, wherein said composition comprises:
(a) 35-55% collagen, 10-30% elastin, less than 0.1% fibronectin by dry weight, an undetectable amount of laminin, and an undetectable amount of glycosaminoglycans; or
(b) 40-70% collagen and 15-25% elastin, less than about 0.1% fibronectin by dry weight, less than about 0.1% laminin by dry weight, less than about 0.1% glycosaminoglycans by dry weight, and an undetectable amount of cytokines, growth factors and/or deoxycholic acid.

4. The composition of any one of claims 1-3, wherein said ECM is placental ECM, optionally human placental ECM.

5. The composition of any one of claims 1-4, wherein said composition is formulated as:
(a) a sheet, optionally wherein said sheet is 1.5 to 2.5 mm thick; or
(b) a particulate; or
(c) a flowable matrix.

6. A method of preparing an ECM composition:
(a) the ECM composition comprising 35-55% collagen by dry weight and 10-30% elastin by dry weight, wherein the method comprises: (i) removing the amnion, chorion, and umbilical cord from a placenta; (ii) placing the remaining placental tissue in a solution comprising 1 M NaCl; (iii) homogenizing the placental tissue; (iv) contacting the placental tissue with a solution comprising 2% sodium deoxycholate; (v) washing the placental tissue with water; (vi) contacting the placental tissue with a solution comprising 1 M sodium hydroxide; (vii) bringing the solution comprising the placental tissue to a neutral pH; and (viii) collecting the placental tissue; or
(b) the ECM composition comprising 40-70% collagen by dry weight and 15-25% elastin by dry weight, wherein the method comprises: (i) obtaining the chorion from a placenta; (ii) scraping and cleaning the chorion; (iii) placing the chorion tissue in solution comprising 0.5 M NaCl; (iv) contacting the chorion with a solution comprising 2% deoxycholic acid or sodium deoxycholate; (v) rinsing the chorion with water; and (vi) grinding and freeze drying; or
(c) the ECM composition comprising 40-70% collagen by dry weight and 15-25% elastin by dry weight, wherein the method comprises: (i) obtaining the chorion from a placenta; (ii) scraping and cleaning the chorion; (iii) placing the chorion in a solution comprising 1.0 M NaCl; (iv) contacting the chorion with a first detergent solution comprising 0.05-0.1% deoxycholic acid or sodium deoxycholate and 3mM-5mM EDTA, following by water rinsing; (iv) contacting the chorion with a second detergent solution comprising 0.05-0.1% deoxycholic acid or sodium deoxycholate, followed by water rinsing; (v) and freeze drying.

7. The method of claim 6, wherein said method results in a composition comprising:
(a) less than about 0.1% fibronectin by dry weight; and/or
(b) an undetectable amount of laminin or less than about 0.1% laminin by dry weight; and/or
(c) an undetectable amount of glycosaminoglycans or less than about 0.1% glycosaminoglycans by dry weight; and/or
(d) an undetectable amount of cytokines, growth factors or deoxycholic acid.

8. The method of claim 7, wherein said method results in a composition comprising:
(a) 35-55% collagen by dry weight, 10-30% elastin by dry weight, less than 0.1% fibronectin by dry weight, an undetectable amount of laminin, and an undetectable amount of glycosaminoglycans; or
(b) 40-70% collagen by dry weight and 15-25% elastin by dry weight, less than about 0.1% fibronectin by dry weight, less than about 0.1% laminin by dry weight, less than about 0.1% glycosaminoglycans by dry weight, and an undetectable amount of cytokines, growth factors and/or deoxycholic acid.

9. The method of any one of claims 6-8, wherein said ECM composition is obtained from human placental tissue.

10. The method of any one of claims 6-9, wherein said composition is formulated as:
(a) a sheet, optionally wherein said sheet is 1.5 to 2.5 mm thick; or
(b) a particulate; or
(c) a flowable matrix.

11. The composition of any one of claim 1-5 for use in a method of treatment.

12. The composition for use in a method according to claim 11, the method comprising administering to a subject, or contacting a subject with the composition, wherein said subject has: an oral lesion, urinary incontinence, vesicoureteral reflux, reflux disease, a disease, disorder, or abnormality that affects one or both vocal cords and/or the larynx, or glottic incompetence.

13. The composition for use in a method according to claim 11, wherein said ECM is used as: a nerve guide, a tendon wrap, a dural replacement.

14. Cosmetic use of the composition of any one of claims 1-5 for treating a cosmetic defect in a subject, the method comprising administering to a subject, or contacting a subject with the composition of any one of claims 1-5.

15. The cosmetic use of claim 14, wherein said cosmetic defect is wrinkles, depressions, creases, stretch marks, scars, hollow eyes, or visible vessels resulting in dark circles around the eyes.

## Patentansprüche

1. Extrazelluläre-Matrix- (EZM-) Zusammensetzung, die Folgendes umfasst:
(a) 35-55 % Kollagen bezogen auf das Trockengewicht und 10-30 % Elastin bezogen auf das Trockengewicht oder
(b) 40-70 % Kollagen bezogen auf das Trockengewicht und 15-25 % Elastin bezogen auf das Trockengewicht.

2. Zusammensetzung nach Anspruch 1, wobei die Zusammensetzung Folgendes umfasst:
(a) weniger als 0,1 % Fibronektin bezogen auf das Trockengewicht; und/oder
(b) eine nicht nachweisbare Menge Laminin oder weniger als etwa 0,1 % Laminin bezogen auf das Trockengewicht; und/oder
(c) eine nicht nachweisbare Menge Glykosaminoglykane oder weniger als etwa 0,1 % Glykosaminoglykane bezogen auf das Trockengewicht; und/oder
(d) eine nicht nachweisbare Menge Cytokine, Wachstumsfaktoren und/oder Desoxycholsäure.

3. Zusammensetzung nach Anspruch 1, wobei die Zusammensetzung Folgendes umfasst:
(a) 35-55 % Kollagen, 10-30 % Elastin, weniger als 0,1 % Fibronektin bezogen auf das Trockengewicht, eine nicht nachweisbare Menge Laminin und eine nicht nachweisbare Menge Glykosaminoglykane; oder
(b) 40-70 % Kollagen und 15-25 % Elastin, weniger als etwa 0,1 % Fibronektin bezogen auf das Trockengewicht, weniger als etwa 0,1 % Laminin bezogen auf das Trockengewicht, weniger als etwa 0,1 % Glykosaminoglykane bezogen auf das Trockengewicht und eine nicht nachweisbare Menge Cytokine, Wachstumsfaktoren und/oder Desoxycholsäure.

4. Zusammensetzung nach einem der Ansprüche 1-3, wobei die EZM plazentare EZM, gegebenenfalls menschliche plazentare EZM, ist.

5. Zusammensetzung nach einem der Ansprüche 1-4, wobei die Zusammensetzung als Folgendes formuliert ist:
(a) als Platte, wobei die Platte gegebenenfalls 1,5 bis 2,5 mm dick ist; oder
(b) in partikulärer Form; oder
(c) als fließfähige Matrix.

6. Verfahren zur Herstellung einer EZM-Zusammensetzung:
(a) wobei die EZM-Zusammensetzung 35-55 % Kollagen bezogen auf das Trockengewicht und 10-30 % Elastin bezogen auf das Trockengewicht umfasst, wobei das Verfahren Folgendes umfasst: (i) Entfernen des Amnions, des Chorions und der Nabelschnur von einer Plazenta; (ii) Platzieren des restlichen Plazentagewebes in eine Lösung, die 1 M NaCl umfasst; (iii) Homogenisieren des Plazentagewebes; (iv) Inkontaktbringen des Plazentagewebes mit einer Lösung, die 2 % Natriumdesoxycholat umfasst; (v) Waschen des Plazentagewebes mit Wasser; (vi) Inkontaktbringen des Plazentagewebes mit einer Lösung, die 1 M Natriumhydroxid umfasst; (vii) Bringen der das Plazentagewebe umfassenden Lösung auf einen neutralen pH; und (viii) Gewinnen des Plazentagewebes; oder
(b) wobei die EZM-Zusammensetzung 40-70 % Kollagen bezogen auf das Trockengewicht und 15-25 % Elastin bezogen auf das Trockengewicht umfasst, wobei das Verfahren Folgendes umfasst: (i) Gewinnen des Chorions von einer Plazenta; (ii) Abschaben und Reinigen des Chorions; (iii) Platzieren des Choriongewebes in eine Lösung, die 0,5 M NaCl umfasst; (iv) Inkontaktbringen des Chorions mit einer Lösung, die 2 % Desoxycholsäure oder Natriumdesoxycholat umfasst; (v) Spülen des Chorions mit Wasser; und (vi) Mahlen und Gefriertrocknen; oder
(c) wobei die EZM-Zusammensetzung 40-70 % Kollagen bezogen auf das Trockengewicht und 15-25 % Elastin bezogen auf das Trockengewicht umfasst, wobei das Verfahren Folgendes umfasst: (i) Gewinnen des Chorions von einer Plazenta; (ii) Abschaben und Reinigen des Chorions, (iii) Platzieren des Chorions in eine Lösung, die 0,1 M NaCl umfasst; (iv) Inkontaktbringen des Chorions mit einer ersten Detergenslösung, die 0,05-0,1 % Desoxycholsäure oder Natriumdesoxycholat und 3 mM bis 5 mM EDTA umfasst, gefolgt von Spülen mit Wasser; (iv) Inkontaktbringen des Chorions mit einer zweiten Detergenslösung, die 0,05-0,1 % Desoxycholsäure oder Natriumdesoxycholat umfasst, gefolgt von Spülen mit Wasser; (v) und Gefriertrocknen.

7. Verfahren nach Anspruch 6, wobei das Verfahren zu einer Zusammensetzung führt, die Folgendes umfasst:
(a) weniger als etwa 0,1 % Fibronektin bezogen auf das Trockengewicht; und/oder
(b) eine nicht nachweisbare Menge Laminin oder weniger als etwa 0,1 % Laminin bezogen auf das Trockengewicht; und/oder
(c) eine nicht nachweisbare Menge Gykosaminoglykane oder weniger als etwa 0,1 % Glykosaminoglykane bezogen auf das Trockengewicht; und/oder
(d) eine nicht nachweisbare Menge Cytokine, Wachstumsfaktoren oder Desoxycholsäure.

8. Verfahren nach Anspruch 7, wobei das Verfahren zu einer Zusammensetzung führt, die Folgendes umfasst:
(a) 35-55 % Kollagen bezogen auf das Trockengewicht, 10-30 % Elastin bezogen auf das Trockengewicht, weniger als 0,1 % Fibronektin bezogen auf das Trockengewicht, eine nicht nachweisbare Menge Laminin und eine nicht nachweisbare Menge Glykosaminoglykane; oder
(b) 40-70 % Kollagen bezogen auf das Trockengewicht und 15-25 % Elastin bezogen auf das Trockengewicht, weniger als etwa 0,1 % Fibronektin bezogen auf das Trockengewicht, weniger als etwa 0,1 % Laminin bezogen auf das Trockengewicht, weniger als etwa 0,1 % Glykosaminoglykane bezogen auf das Trockengewicht und eine nicht nachweisbare Menge Cytokine, Wachstumsfaktoren und/oder Desoxycholsäure.

9. Verfahren nach einem der Ansprüche 6-8, wobei die EZM-Zusammensetzung aus menschlichem Plazentagewebe erhalten wird.

10. Verfahren nach einem der Ansprüche 6-9, wobei die Zusammensetzung als Folgendes formuliert ist:
(a) als Platte, wobei die Platte gegebenenfalls 1,5 bis 2,5 mm dick ist; oder
(b) in partikulärer Form; oder
(c) als fließfähige Matrix.

11. Zusammensetzung nach einem der Ansprüche 1-5 zur Verwendung in einem Behandlungsverfahren.

12. Zusammensetzung zur Verwendung in einem Verfahren nach Anspruch 11, wobei das Verfahren die Verabreichung der Zusammensetzung an ein Individuum oder das Inkontaktbringen des Individuums damit umfasst, wobei das Individuum Folgendes aufweist: eine orale Läsion, Harninkontinenz, vesikouretheralen Reflux, Refluxkrankheit, eine Erkrankung, Störung, eine Anomalie, die ein oder beide Stimmbänder und/oder den Kehlkopf betrifft, Glottis-Inkompetenz.

13. Zusammensetzung zur Verwendung in einem Verfahren nach Anspruch 11, wobei die EZM als Folgendes verwendet wird: Nervenleitung, Sehnenumwicklung, Duraersatz.

14. Kosmetische Anwendung der Zusammensetzung nach einem der Ansprüche 1-5 zur Behandlung eines kosmetischen Defekts bei einem Individuum, wobei das Verfahren das Verabreichen einer Zusammensetzung nach einem der Ansprüche 1-5 an das Individuum oder das Inkontaktbringen eines Individuums damit umfasst.

15. Kosmetische Anwendung nach Anspruch 14, wobei der kosmetische Defekt Falten, Vertiefungen, Fältchen, Dehnungsstreifen, Narben, hohle Augen oder sichtbare Gefäße, die zu dunklen Ringen um die Augen führen, sind.

## Revendications

1. Composition de matrice extracellulaire (ECM) comprenant :
(a) de 35 à 55 % de collagène en poids sec et de 10 à 30 % d'élastine en poids sec, ou
(b) de 40 à 70 % de collagène en poids sec et de 15 à 25 % d'élastine en poids sec.

2. Composition selon la revendication 1, dans laquelle ladite composition comprend :
(a) moins de 0,1 % de fibronectine en poids sec ; et/ou
(b) une quantité indétectable de laminine, ou moins d'environ 0,1 % de laminine en poids sec ; et/ou
(c) une quantité indétectable de glycosaminoglycanes, ou moins d'environ 0,1 % de glycosaminoglycanes en poids sec ; et/ou
(d) une quantité indétectable de cytokines, de facteurs de croissance et/ou d'acide désoxycholique.

3. Composition selon la revendication 1, dans laquelle ladite composition comprend :
a) de 35 à 55 % de collagène, de 10 à 30 % d'élastine, moins de 0,1 % de fibronectine en poids sec, une quantité indétectable de laminine et une quantité indétectable de glycosaminoglycanes ; ou
(b) de 40 à 70 % de collagène et de 15 à 25 % d'élastine, moins d'environ 0,1 % de fibronectine en poids sec, moins d'environ 0,1 % de laminine en poids sec, moins d'environ 0,1 % de glycosaminoglycanes en poids sec, et une quantité indétectable de cytokines, de facteurs de croissance et/ou d'acide désoxycholique.

4. Composition selon l'une quelconque des revendications 1 à 3, dans laquelle ladite ECM est une ECM placentaire, facultativement une ECM placentaire humaine.

5. Composition selon l'une quelconque des revendications 1 à 4, dans laquelle ladite composition est formulée sous la forme :
(a) d'une feuille, facultativement dans laquelle ladite feuille présente une épaisseur de 1,5 à 2,5 mm ; ou
(b) d'une matière particulaire ; ou
(c) d'une matrice fluide.

6. Procédé de préparation d'une composition ECM :
(a) la composition ECM comprenant de 35 à 55 % de collagène en poids sec et 10 à 30 % d'élastine en poids sec, dans lequel le procédé comprend les étapes consistant à :
(i) retirer l'amnion, le chorion et le cordon ombilical à partir d'un placenta ;
(ii) placer le tissu placentaire restant dans une solution comprenant 1 M de NaCl ;
(iii) homogénéiser le tissu placentaire ;
(iv) mettre en contact le tissu placentaire avec une solution comprenant 2 % de désoxycholate de sodium ;
(v) laver le tissu placentaire avec de l'eau ;
(vi) mettre en contact le tissu placentaire avec une solution comprenant 1 M d'hydroxyde de sodium ;
(vii) amener la solution comprenant le tissu placentaire à un pH neutre ; et
(viii) recueillir le tissu placentaire ;
(b) la composition ECM comprenant de 40 à 70 % de collagène en poids sec et 15 à 25 % d'élastine en poids sec, dans lequel le procédé comprend les étapes consistant à :
(i) obtenir le chorion à partir d'un placenta ;
(ii) gratter et nettoyer le chorion ;
(iii) placer le tissu chorionique dans une solution comprenant 0,5 M de NaCl ;
(iv) mettre en contact le chorion avec une solution comprenant 2 % d'acide désoxycholique ou de désoxycholate de sodium ;
(v) rincer le chorion à l'eau ;
(vi) broyer et lyophiliser ; ou
(c) la composition ECM comprenant de 40 à 70 % de collagène en poids sec et de 15 à 25 % d'élastine en poids sec, dans lequel le procédé comprend les étapes consistant à :
(i) obtenir le chorion à partir d'un placenta ;
(ii) gratter et nettoyer le chorion ;
(iii) placer le chorion dans une solution comprenant 1,0 M de NaCl ;
(iv) mettre en contact le chorion avec une première solution détergente comprenant de 0,05 à 0,1 % d'acide désoxycholique ou de désoxycholate de sodium et de 3 mM à 5 mM d'EDTA, suivie d'un rinçage à l'eau ;
(iv) mettre en contact le chorion avec une seconde solution détergente comprenant de 0,05 à 0,1 % d'acide désoxycholique ou de désoxycholate de sodium, suivi d'un rinçage à l'eau ;
(v) et lyophiliser.

7. Procédé selon la revendication 6, dans lequel ledit procédé résulte en une composition comprenant :
(a) moins d'environ 0,1 % de fibronectine en poids sec ; et/ou
(b) une quantité indétectable de laminine ou moins d'environ 0,1 % de laminine en poids sec ; et/ou
(c) une quantité indétectable de glycosaminoglycanes ou moins d'environ 0,1 % de glycosaminoglycanes en poids sec ; et/ou
(d) une quantité indétectable de cytokines, de facteurs de croissance ou d'acide désoxycholique.

8. Procédé selon la revendication 7, dans lequel ledit procédé résulte en une composition comprenant :
a) de 35 à 55 % de collagène en poids sec, de 10 à 30 % d'élastine en poids sec, moins de 0,1 % de fibronectine en poids sec, une quantité indétectable de laminine et une quantité indétectable de glycosaminoglycanes ;
(b) de 40 à 70 % de collagène en poids sec et de 15 à 25 % d'élastine en poids sec, moins d'environ 0,1 % de fibronectine en poids sec, moins d'environ 0,1 % de laminine en poids sec, moins d'environ 0,1 % de glycosaminoglycanes en poids sec, et une quantité indétectable de cytokines, de facteurs de croissance et/ou d'acide désoxycholique.

9. Procédé selon l'une quelconque des revendications 6 à 8, dans lequel ladite composition EMC est obtenue à partir de tissu placentaire humain.

10. Procédé selon l'une quelconque des revendications 6 à 9, dans lequel ladite composition est formulée sous la forme :
(a) d'une feuille, facultativement dans laquelle ladite feuille présente une épaisseur de 1,5 à 2,5 mm ; ou
(b) d'une matière particulaire ; ou
(c) d'une matrice fluide.

11. Composition selon l'une quelconque des revendications 1 à 5 à utiliser dans un procédé de traitement.

12. Composition à utiliser dans un procédé selon la revendication 11, le procédé comprenant une administration à un sujet, ou une mise en contact d'un sujet avec la composition, dans laquelle ledit sujet présente : une lésion orale, une incontinence urinaire, un reflux vésico-urétral, une maladie de reflux, une maladie, un trouble ou une anomalie qui affecte l'un ou les deux parmi les cordes vocales et/ou le larynx, ou une incompétence glottique.

13. Composition à utiliser dans un procédé selon la revendication 11, dans laquelle ladite ECM est utilisée sous la forme : un guide de nerf, une enveloppe tendineuse, un substitut dural.

14. Utilisation cosmétique de la composition selon l'une quelconque des revendications 1 à 5 pour le traitement d'un défaut cosmétique chez un sujet, le procédé comprenant une administration à un sujet, ou une mise en contact d'un sujet avec la composition selon l'une quelconque des revendications 1 à 5.

15. Utilisation cosmétique selon la revendication 14, dans laquelle ledit défaut cosmétique est constitué par des rides, des enfoncement, des plis, des vergetures, des cicatrices, des yeux creux ou des vaisseaux visibles résultant en des cernes autour des yeux.
